# EUROPEAN PATENT SPECIFICATION

(11) **EP 0 668 720 B1**
(45) Date of publication and mention of the grant of the patent: **14.08.2002**
(21) Application number: 94901405.4
(22) Date of filing: 12.11.1993
(51) Int. Cl.: A61L 29/16, A61K 31/65, A61K 31/198

(54) **Coating of minocyclin and EDTA for medical devices**
Minocyclin und EDTA enthaltende Beschichtungszusammensetzung für Medizinische Geräte
Revêtement à base de minocycline et de l'EDTA pour dispositifs médicaux

(30) Priority: 12.11.1992 US 975486
(43) Date of publication of application: 30.08.1995
(73) Proprietor: BOARD OF REGENTS THE UNIVERSITY OF TEXAS SYSTEM, Austin, Texas 78701 (US)
(72) Inventor: RAAD, Issam, Houston, TX 77071 (US); SHERERTZ, Robert, J., Winston-Salam, NC 27106 (US)
(74) Representative: Grünecker, Kinkeldey, Stockmair & Schwanhäusser Anwaltssozietät
(86) International application number: PCT/US93/10893
(87) International publication number: WO 94/10838

(56) References cited:
- EP-A- 0 122 709
- US-A- 5 202 449
- ROOT JENNIFER L ET AL: "Inhibitory effect of disodium EDTA upon the growth of Staphylococcus epidermidis in vitro: Relation to infection Prophylaxis of Hickman Catheters" ANTIMICROBIAL AGENTS AND CHEMOTHERAPY, vol. 32, no. 11, November 1988, pages 1627-1631, XP002107990
- WEISER R ET AL: "In vitro reversal of antibiotic resistance by ethylenediamine tetraacetic acid" NATURE, vol. 219, 28 September 1968, pages 1365-1366, XP002107991 London, GB
- MCMURRY LAURA M. ET AL: "Susceptible Escherichia coli cells can actively excrete tetracyclines" ANTIMICROBIAL AGENTS AND CHEMOTHERAPY, vol. 24, no. 4, October 1983, pages 544-551, XP002107992
- EVANS RC, HOLMES CJ: 'Effect of Voncomycin hydrochloride on Staphylococcus Epidermidis biofilm associated with silicon elastomer.' ANTIMICROBIAL AGENTS AND CHEMOTHERAPY vol. 31, no. 6, 1987, pages 889 - 894
- FARBER BF, KAPLAN MH, CLOGSTON AG: 'Staphyloccus epidermidis extracted slime inhibits the antimicrobial action on glycopeptide antibiotics' JOURNAL OF INFECTIOUS DISEASES vol. 161, 1990, pages 37 - 40
- NICKEL JC, RUSESKA I, WRIGHT JB, COSTERTON JW: 'Tobramycin resistance of Pseudomonas aeruginosa cells growing as a biofilm on urinary catheter material' ANTIMICROBIAL AGENTS AND CHEMOTHERAPY vol. 27, no. 4, 1985, pages 619 - 624
- PEARSON ML: 'Guideline for prevention of intravascular device related infections.' INFECTION CONTROL AND HOSPITAL EPIDEMIOLOGY vol. 17, 1996, pages 438 - 473

## Description

### BACKGROUND OF THE INVENTION

### 1. FIELD OF THE INVENTION

The present invention relates to indwelling medical articles, such as catheters, which are flushed or coated with a microbial-inhibiting pharmaceutical preparation. The invention also relates to pharmaceutical preparations useful in maintaining catheter patency and preventing infection. Methods of using the pharmaceutical preparation of the invention in the management and maintenance of a vascular catheter are also related to the present disclosure.

### 2. BACKGROUND OF THE RELATED ART

Indwelling medical devices including vascular catheters have become essential in the management of hospitalized or chronically ill patients. Unfortunately, vascular catheters have become the major source for hospital-acquired sepsis. Hence, the benefit derived from indwelling medical devices such as vascular catheters is often upset by infectious complications. Thrombotic occlusions of the lumen of central venous catheters (CVC) is another complication that will often lead to the removal of catheters.

The current standard care of catheters includes flushing the lumen of the catheter with heparin. However, heparin has no antimicrobial activity. Thus, infections, as well as thrombotic occlusion, continue to occur frequently despite the prophylactic use of heparin flushes. Knowledge of the pathogenesis and microbiology of central venous catheter-related infections is essential in order to provide effective prevention for this problem. Three essential factors must be considered in controlling for catheter colonization by infectious microbes. The first is controlling the availability of microorganisms that adhere to the inert catheter surface. Such microorganisms typically include staphylococci and candida. The second is controlling the production of a polysaccharide by adherent known as organisms fibrous glycocalyx. Production of the glycocalyx is essential for the adherence and integrity of these organisms. The third is control of the formation of a thrombin sheath by the host, which acts to engulf the catheter. The thrombin sheath provides the microorganisms a sticky substrate for enhanced adherence to the catheter, and thus, continued colonization and infection at the catheter site. The present inventors herein disclose an M-EDTA solution unique in its ability to inhibit all three of these essential conditions, and thus provide effective methods for controlling catheter-related infection and onset thereof.

*Staphylococcus epidermidis* and *S. aureus* account for 75% of CVC related infections. *Candida* species account for another 10% to 15% of such infections. The use of antistaphylococcal antibiotics to prevent these infections has been found to reduce CVC related bacterial infections, but only at the expense of the occurrence of higher rates of fungal (*Candida*) infections. The fibrous glycocalyx material produced by staphylococci and Candida helps these organisms adhere and stick to catheter surfaces, thus exacerbating the problem of eliminating these types of infections after they have become established. These microbial biofilm layers are made of a fibrous glycocalyx material primarily polysaccharide in nature. The protective sheath provided by the glycocalyx at the infected site effectively prevents the elimination and treatment of these infections. Preparations effective for destroying such a glycocalyx would, therefore, provide a solution for treating established catheter infections, particularly where a glycocalyx is already formed.

Compositionally distinct glycocalyx material is produced by a variety of different organisms. For example, a mostly protein glycocalyx produced by *Hymenolepis diminuta* (tapeworm) is reportedly eliminated upon treatment with 0.02 M-EDTA or 3 M KCl¹². This material, however, is compositionally distinct from the material of the glycocalyx formed by organisms that typically colonize and cause catheter infection. For example, the glycocalyx of several staphylococcus species comprise primarily polysaccharides with only low to nondeductible levels of protein¹³ (Tojo *et al*. at pg. 716, Table 1). Glycocalyx of microorganisms common to catheter infection are thus compositionally distinct from the glycocalyx of such organisms as the tapeworm, *Hymenolepis diminuta*. A pharmaceutical preparation effective for reducing or eliminating glycocalyx of infectious microorganisms typically associated with catheter colonization and infection has yet to be identified.

Infectious microorganisms will typically embed themselves in the protective layer of the glycocalyx, thus providing a shield or hiding place that protects staphylococci and fungi from the activity of the host's phagocytic cells. An agent or composition that would dissolve or prevent the glycocalyx (biofilm) formation of these clinically important pathogens would thus provide a major breakthrough in the prevention of typical catheter-related *Staphylococcal* and *Candida* infections.

There has also been observed to be a correlation between thrombogenesis and infection. Indwelling vascular catheters are typically engulfed by a fibrin sheath that subsequently acts to cover the internal and external surfaces of a catheter. This fibrin sheath provides such organisms as *Staphylococci* and *Candida,* with an enhanced adherence capacity to the catheter surface. Unlike these particular microbes, gram-negative bacilli do not adhere well to fibrin and fibronectin. A composition that halts fibrin formation would thus be particularly useful in halting the colonization of these microbes at indwelling catheter sites.

Intraluminal colonization through a catheter hub also constitutes a prelude to catheter-related infections and septicemias in long-term CVC. The inventors study presented herein of long-term CVC patients (studied by quantitative catheter culture) demonstrates that CVC's with positive cultures as well as matched negative controls evidenced colonization (as quantitated by EM) and biofilm formation of the internal surface at least twice greater than that of external surface with catheters that stayed longer than 10 days in place. This data is from nontunneled, noncuffed percutaneous CVC. For tunneled CVCs (Hickman/Broviac) and ports, internal colonization was even more prominent. The development of an anticoagulant pharmaceutical preparation effective against staphylococci, fungi, and polysaccharide-rich glycocalyx formation, such as that observed during microorganism intraluminal colonization of a catheter hub, would provide a solution to the treatment and elimination of thrombogenesis and the septicemia associated with long-term CVC.

EDTA is an anticoagulant used in blood collection, and is also described as having an antibacterial and antistaphylococcal effect (alone or in combination)¹⁻³. Root⁹ describsd the efficacy of EDTA in vascular catheters as an antibacterial agent as compared to heparin alone and as compared to a vancomycin-heparin preparation *in vitro*. These investigators found EDTA to have some bacteriocidal activity. However, no remedy or suggestion for treatment of how a microbial glycocalyx, such as that observed during *in vivo* catheter-related infection, was observed or described.

Glycopeptide antibiotics (vancomycin and teicoplanin) are active against staphylococci *in vitro* and in tissue. Vancomycin is currently the standard antibiotic used in the treatment of *Staphylococcus epidermidis* and resistant *Staphylococcus aureus.* However, these antibiotics are not active against adherent staphylococci embedded in a biofilm layer, such as glycocalyx. In addition, while catheter flushing with such agents may destroy initial strains of staphylococci, such is typically not effective against tolerant and resistant strains of the organism that continue the infection. Flushing with an antibiotic preparation, such as vacomycin, would therefore be of only limited therapeutic value against catheter infection.

The ideal prophylactic agent or treatment for catheter maintenance would inhibit or eliminate the formation of polysaccharide-rich glycocalyx of microorganisms and would also inhibit staphylococci and fungi infectious growth.

It is an object of the invention to provide both an antistaphylococcal and antifungal (anti-*Candida*) active agent effective against free-floating as well as adherent organisms embedded in biofilm, as well as to provide an anticoagulant agent and/or method that would prevent or inhibit the formation of polysaccharide-rich fibrous glycocalyx biofilm layer. Such a pharmaceutical agent would optimally prevent thrombotic occlusion of the catheter lumen and prevent thrombin formation. Additional objects of the invention include providing an agent that could be given intraluminally without a toxicity concern to humans and to provide a method that would kill adherent *Staphylococci* and *Candida.* Such methods would preferably not include the use of pharmaceutical agents typically used to treat non-catheter related infections (such as Vancomycin, Ampho B, or Azoles).

The present invention demonstrates that a mixture of minocycline/disodium EDTA (referred to as M-EDTA) does fulfill all of the listed objects.

### SUMMARY OF THE INVENTION

The present invention provides a unique and effective pharmaceutical preparation that includes minocycline and EDTA. These mixtures are shown to be effective for maintaining the patency of a medical device, such as a catheter, *in vivo.* As used in the description of the present invention, "patency" is defined as the state of being freely open or patulous, particularly a catheter opening that is unobscured by the formation of a microorganismal polysaccharide-rich fibrous glycocalyx. In a preferred embodiment, minocycline and EDTA are included in the disclosed preparations in pharmacologically effective amounts together in a pharmacologically acceptable carrier solution.

The EDTA of the preparation provides potent glycocalyx inhibiting potential, while the minocycline at high concentrations has a fungicidal effect and a unique ability to penetrate a polysaccharide-rich glycocalyx biofilm layer. The combination of minocycline-EDTA thus provides a uniquely effective anticoagulant, anti-microbial, glycocalyx inhibiting, antibacterial and antifungal agent for the prevention of thrombogenesis, microbial adherence and device-related infections.

The inventors demonstrate that minocycline has the surprising activity of penetrating a polysaccharide-rich microbial glycocalyx biofilm layer at least 6-fold more effectively than vancomycin. The inventors also demonstrate that EDTA effectively prevents and dissolves polysaccharide-rich microbial glycocalyx formation at implanted catheter sites *in vivo.*

In still another aspect of the invention, methods for using minocycline for the manufacture of an antimicrobial agent are provided. Minocycline is demonstrated to kill adherent staphylococci (embedded in glycocalyx - Example 4), and to be superior to vancomycin as an antibacterial agent (Table 3) in actual *in vivo* trials.

For use in maintaining catheter patency, the pharmaceutical preparation of the invention may be efficaciously used in conjunction with virtually any device in which a clear path for the flow of biological fluids is necessary. For example, such devices include a central venous catheter, a peripheral intervenous catheter, an arterial catheter, a Swan-Ganz catheter, a hemodialysis catheter, an umbilical catheter, a percutaneous nontunneled silicone catheter, a cuffed tunneled central venous catheter as well as a subcutaneous central venous port.

In a preferred embodiment, a solution of the M-EDTA may be prepared containing a concentration of between 10 to 100 mg/ml EDTA (preferably between 20 to 60 mg/ml) and between 0.001 to 100 mg/ml minocycline (preferably between 2 and 9 mg/ml). Most preferably, the preparation as a device or catheter flushing solution includes 30 mg/ml EDTA and 3 mg/ml minocycline. The minocycline may preferably be prepared as a reconstituted minocycline preparation, such as from a 100 mg vial of minocycline commercially available (Minocin Intravenous, Lederle (Carolina, Puerto Rico). The carrier solution, by way of example, may comprise saline, preferably sterile saline, available from commercial sources.

In another aspect of the invention, a catheter flushing solution is provided. Most preferably, the catheter flushing solution comprises a glycocalyx inhibiting concentration of EDTA and minocycline in a pharmaceutically acceptable carrier solution. More specifically, the concentration of EDTA in the flushing preparation is between 1 to 100 mg/ml with the concentration of minocycline being between 0.001 to 100 mg/ml in the preparation. Most preferably, the catheter flushing solution includes 30 mg/ml EDTA and 3 mg/ml minocycline.

By way of example, the carrier solution of the flushing preparation is saline, preferably sterile saline. The catheter flushing preparation of the present invention may advantageously be used to inhibit the formation of polysaccharide-rich glycocalyx. In this manner, infections characterized by the presence of a polysaccharide-rich glycocalyx, such as a glycocalyx which is at least 50% polysaccharide by composition, may be effectively treated and/or eliminated.

A "glycocalyx inhibiting concentration" is defined for purposes of describing the present invention as a concentration of minocycline, EDTA or a combination thereof effective to degrade, dissolve, or otherwise inhibit polysaccharide-rich glycocalyx formation. By way of example, a polysaccharide-rich glycocalyx is characteristic of established staphylococcal infections of *S*. *aureus* and *S. epidermidis.*

Another aspect of the present invention provides a method of preparing a biofilm-resistant device, as well as bio-film resistant devices that are coated with minocycline and EDTA. The method in one embodiment comprises coating a device with a coating preparation minocycline and EDTA. While the method may be used to coat virtually any surface where glycocalyx formation is to be desirably inhibited, use of the method in coating a catheter device is particularly envisioned. It is anticipated that the method will provide a device resistant to polysaccharide-rich (at least about 50% polysaccharide) glycocalyx formation, such as that characteristic of Staphylococci. Any of a variety of catheters may be treated or coated according to the described method employing coating techniques well known to those of skill in the art. By way of example, catheters that may be prepared and treated according to the invention include a central venous catheter and a triple lumen catheter.

In a most preferred embodiment, the method for preparing a biofilm-resistant medical device using a pharmaceutical preparation of minocycline and EDTA comprises preparing a solution of minocycline and sodium EDTA, most preferably in a biocompatible adherent coating carrier solution, and treating the surface of the medical device of interest with the solution for a period of time sufficient to allow the formation of a film of the minocycline and EDTA, to the surface of the device. Most preferably, the method is to be used in preparing a biofilm-resistant coating on the surface of a catheter.

Alternatively, a coating of the M-EDTA preparation may be provided at the surface of a device by first treating the surface of the device with a surfactant. Such surfactants include, by way of example, tridodecylmethyl ammonium chloride (TDMACC). Commercially available catheters with such a surfactant already included on the surface are the Bioguard Cook catheters. Accordingly, a device including a surface surfactant may be immersed in a solution of minocycline, EDTA or M-EDTA for a period of time sufficient to allow the solution to permeate the device surface. By way of example, the present inventors have found that immersion of a device in a solution containing 60 mg minocycline and 60 mg EDTA/ml for about 15 minutes at room temperature is sufficient to provide a coating of the solution to the surface of the device. The device is preferably air dried and gas sterilized prior to use.

As used in the description of the present invention, a "biofilm-resistant" device or surface is defined as a surface or device that will prevent the adherence or growth of organisms that produce polysaccharide-rich (about 50% or greater polysaccharide) formations, such as those formations generally referred to as a glycocalyx. Organisms that produce such a glycocalyx include the Staphylococcal organisms, particularly the S. aureus and S. epidermidis species. However, any organism that produces a polysaccharide-rich material would be equally inhibited by the herein described devices, surfaces, pharmaceutical preparations and methods.

The present methods are particularly efficacious for inhibiting polysaccharide-rich glycocalyx formation at a catheter port. The method in one embodiment comprises flushing the catheter periodically with a preparation including a glycocalyx-inhibiting concentration of at least one of EDTA and minocycline, pharmacologically acceptable carrier solution. In one aspect of the method, the glycocalyx-inhibiting concentration of EDTA in the flushing solution is between 0.01 mg/ml and 100 mg/ml. The most preferred concentration of EDTA is 30 mg/ml. According to one aspect of the described method, the catheter may be described as a tunneled catheter or an untunneled catheter. As part of a catheter maintenance regimen, the catheter most preferably is to be flushed with the aforedescribed preparation at least once every 24 to 48 hours.

Particularly stated, the method for eliminating microbial glycocalyx formation, particularly polysaccharide-rich (Staphylococcal) glycocalyx formation, at a catheter lumen comprises preparing a solution comprising minocycline and EDTA in a carrier solution to provide an M-EDTA preparation, and flushing the catheter with a therapeutically effective amount of the M-EDTA preparation sufficient to reduce a polysaccharide glycocalyx. In one embodiment, the M-EDTA preparation includes a concentration of minocycline of between 0.001 to 100 mg/ml (preferably between 2 and 9 mg/ml) and between 10 to 100 mg/ml (preferably between 20 to 60 mg/ml) EDTA. The therapeutically effective amount of the aforedescribed M-EDTA preparation would, therefore, constitute between 1 to 10 ml (preferably 2 to 3 ml) of the aforedescribed solution.

According to the method, a volume of 3 ml of the aforedescribed M-EDTA preparation containing 30 mg/ml EDTA and 3 mg/ml minocycline is used per flush of a catheter or device, depending on the voume of the catheter or device being monitored. For example, a standard size tunneled CVC catheter (Hickman/Brovia) is to be flushed periodically at least once every 24 hours to 48 hours with between 2 to 3 ml of the M-EDTA preparation. In a preferred aspect of the method, the catheter is to be flushed more frequently, such as at least once every 4 hours with the herein described preparations of M-EDTA.

The M-EDTA preparation will remain therapeutically effective for use as a catheter-flushing agent stored at a refrigerated temperature for at least 1 month after formulation. In use, the M-EDTA solution should be brought to room temperature before use on an animal or patient.

The aforedescribed preparations have been found effective in preventing the adherence and colonization of catheter surfaces by *S*. *aureus, S. epidermidis*, and fungi, as well as effective in both treating and eliminating already formed glycocalyx formations of these infectious organisms.

The anti-microbial resistant medical devices of the present invention are further described as a medical device that is processed to include a coating of a combination of M-EDTA. Such a coating would be particularly efficacious for medical devices that include at least one opening or portal, as the coating is shown by the present inventors to be effective for maintaining the patency of such medical device openings as that characteristic of an indwelling catheter *in vivo*. Coating solutions of the M-EDTA include 60 mg minocycline and 60 mg EDTA per ml.

The following abbreviations are used in the description of the present invention:
- CVC =: Central Venous Catheters
- MRD =: Modified Robbins Device
- M-EDTA =: minocycline-EDTA mixture
- D₁₀/W =: 10% Dextrose and Water

### BRIEF DESCRIPTION OF THE DRAWINGS

**Figure 1 -** Scanning electron microscopy picture showing staphylococci and biofilm from a control catheter segment that was exposed to slime producing *S. epidermidis* and later immersed in Dextrinase for 24 hours. (Example 6 results.)
**Figure 2 -** Scanning electron microscopy picture showing a layer of biofilm from a catheter segment that exposed to slime producing *S*. *epidermidis* and later immersed in Urokinase for 24 hours. (Example 6 results.)
**Figure 3 -** Scanning electron microscopy picture showing a representative clear surface from a catheter segment exposed to slime producing *S*. *epidermidis* and later immersed in EDTA for 24 hours. The white particles are dust particles. (Example 6 results.)
**Figure 4 -** A scanning electron micrograph at high magnification showing coccal forms in a biofilm layer from a catheter segment coated with control solution (saline) and later exposed to slime producing *S*. *epidermidis.* (Example 7 results.)
**Figure 5 -** Lower magnification from a different area of the catheter of Figure 4 showing coccal forms and biofilm. (Example 7 results.)
**Figure 6 -** Micrograph of a catheter surface pretreated with dextrinase. A scanning electron micrograph picture showing a catheter surface pretreated or coated with dextrinase, upon exposure to staphylococci. The micrograph shows a thick biofilm layer with many coccal formations. These coccal formations are indicative of staphylococcal colonization.
**Figure 7 -** Electron micrograph demonstrates formation of fibrous glycocalyx on the surface of a control (saline-treated) catheter segment - before flushing with saline for 4 hours.
**Figure 8 -** Electron micrograph demonstrates presence of deranged fibrous glycocalyx on the surface of a minocycline coated catheter segment - before flushing with saline for 4 hours.
**Figure 9 -** Electron micrograph demonstrates fibrous glycocalyx on the surface of a control (saline-treated) catheter segment - after flushing with saline for 4 hours.
**Figure 10 -** Electron micrograph demonstrating fibrous glycocalyx on the surface of a minocycline-coated catheter segment - after flushing with saline for 4 hours.
**Figure 11 -** EDTA Stability (A) and Minocycline Stability (B).

### DETAILED DESCRIPTION OF THE PREFERRED EMBODIMENTS

The present invention provides pharmaceutically effective formulations of minocycline, and EDTA. These formulations have been found by the present inventors to be particularly useful in preventing the formation of the "biofilm" or polysaccharide-rich glycocalyx that typically accompanies microbial surface colonization. In particular, the formulations are most effective in breaking down polysaccharide-rich glycocalyx formations and in inhibiting their formation. This feature makes the preparations of the present invention potentially useful in the treatment of staphylococcal infections where a polysaccharide-rich glycocalyx has formed or may potentially be formed, as well as in the prevention and treatment of *Staphylococcal* and *Candida* infection.

The present invention also provides M-EDTA-treated or coated medical devices, such as catheters, that prevent staphylococcal or fungal colonization.

The minocycline (M) used in the studies described in the present disclosure was obtained from Lederle (Minocin® (intravenous, 100 mg, Carolina, Puerto Rico). The disodium-EDTA used in the studies described in the present disclosure was obtained from Abbott Co. (Endrate® (Intravenous 150 mg/ml) Chicago, IL). A Modified Robbin's Device, a screening tool customarily used and accepted as predictive of catheter use in humans^{15,16}, was used in the present study of the M-EDTA pharmaceutical preparations described. The model was constructed at M.D. Anderson Cancer Center in Houston, Texas.

The following agents were used in the studies disclosed herein:

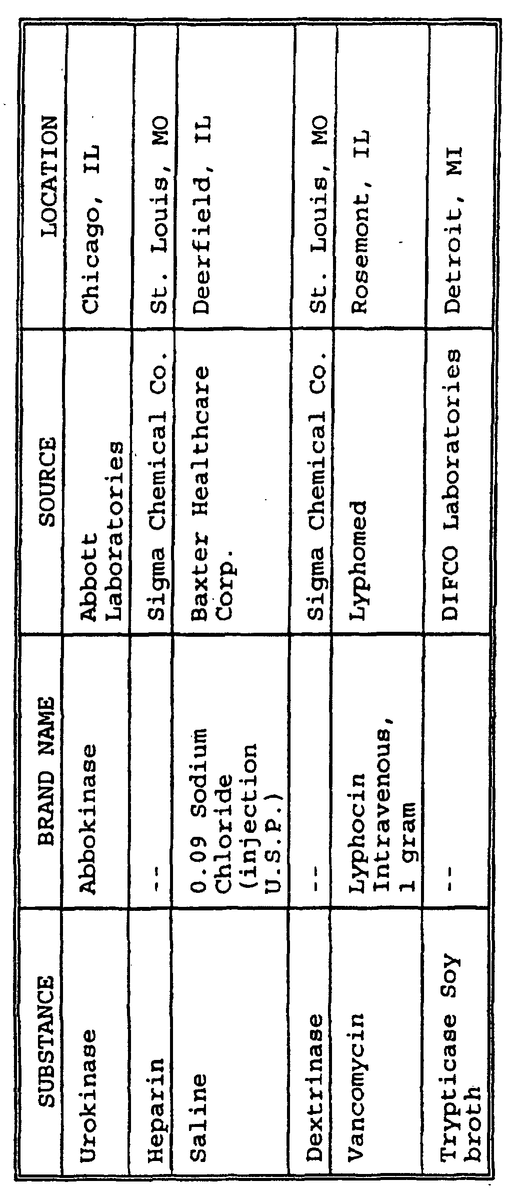

### EXAMPLE 1 - PREPARATION OF M-EDTA PHARMACEUTICAL PREPARATION

The present example provides a detailed description of how the M-EDTA pharmaceutical preparation was prepared for use as a catheter or medical device flushing solution. EDTA was obtained from Sigma. Minocycline was obtained from Lederle.

The M-EDTA solution was prepared as follows so as to achieve a concentration of 3 mg/ml minocycline and 30 mg/ml EDTA in a sterile saline solution. Separate solutions of EDTA (60 mg/ml) and minocycline (3 mg/ml) were prepared in saline. The EDTA was reconstituted from 200 mg/ml Edetate Calcium Disodium (Versenste®, 3M Riker, Northridge, CA) or reconstituted from Edetate Disodium (150 mg/ml parenteral concentrate (Endtrate®, Abbott, Chicago, IL, or Disotate®, Forest, Maryland Heights, MO). Alternatively, the 60 mg/ml of EDTA could be reconstituted from EDTA powder (Sigma Chemical Co., St. Louis, MO). Minocycline was obtained from Lederle and combined with a volume of saline sufficient to constitute 3 mg/ml minocycline.

The 6 mg/ml minocycline and 60 mg/ml EDTA solutions were mixed in equal volumes to constitute a 3 mg minocycline and 30 mg EDTA/ml solution. The solution was then brought to a physiologically acceptable pH of about 7.4. The solution was stored in a sterile container.

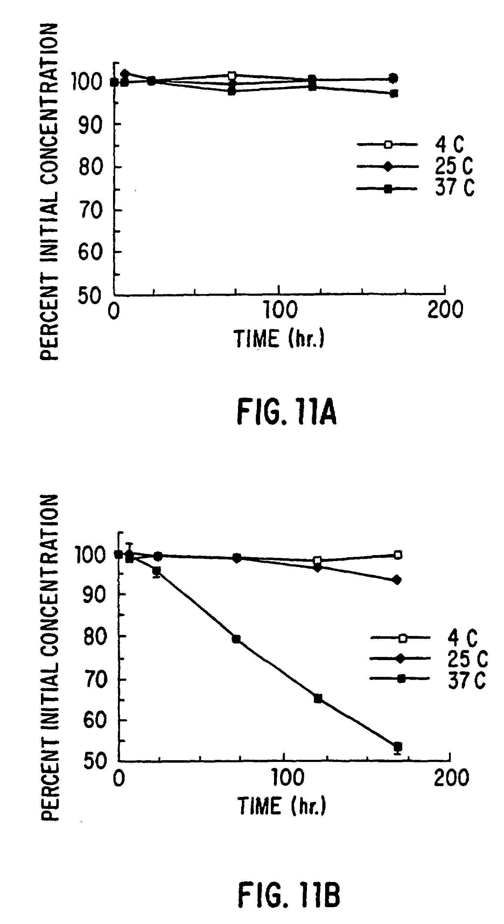

Once formulated, the M-EDTA may be stored refrigerated at 4°C until use. It is contemplated that so formulated, the solution will remain chemically stable and pharmacologically active for at least 1 month at 4°C. The preparation is also very stable at room temperature (37°C) for at least 72 hours (Table 1 and Figure 11). The preparation should be at room temperature before administration to a patient or animal.

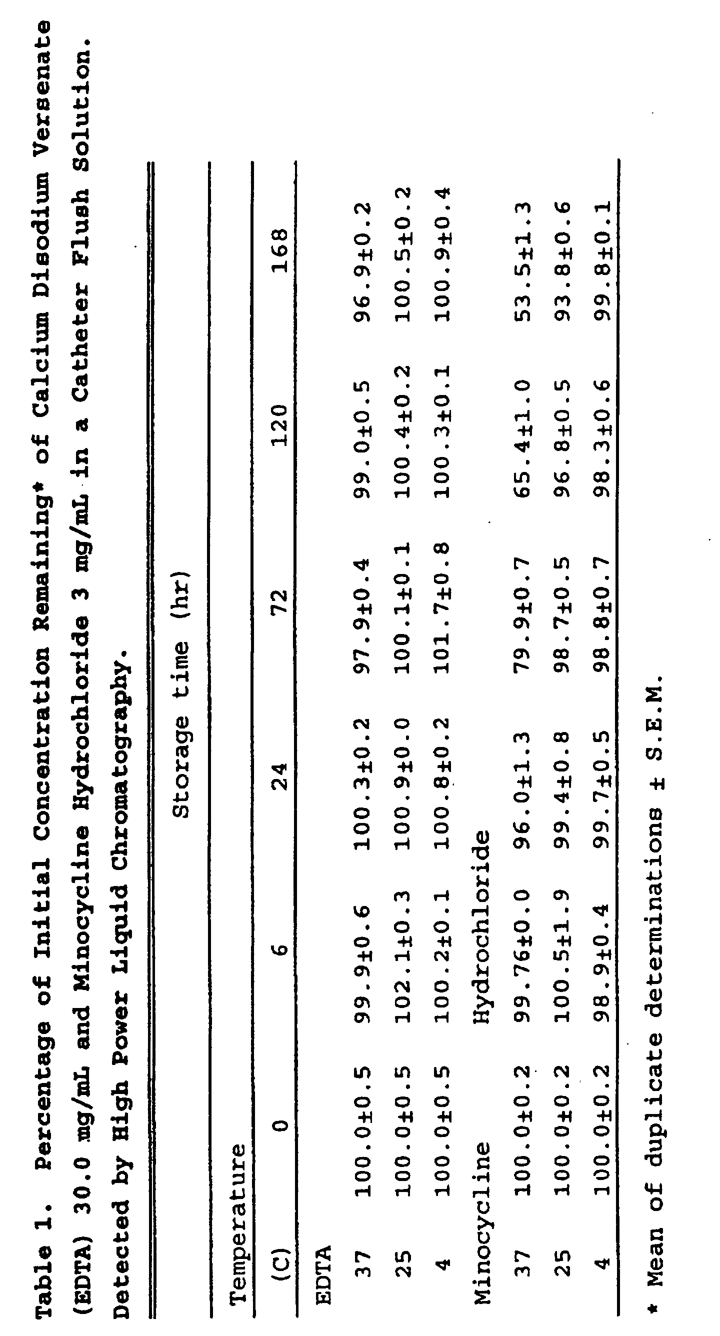

### EXAMPLE 2

### THE MODIFIED ROBBINS DEVICE MODEL

The present example is provided to describe the study model employed for illustrating the antimicrobial and therapeutic utility of the minocycline and EDTA preparations of the present invention.

An *in vitro* model, the Modified Robbin's Device (MRD), was used to study the formation of biofilm and colonization of catheter segments of *S. epidermidis.* This is a well established model that is described in Nickel *et al*.¹⁵ and Evans and Holmes¹⁶, and provides a study model recognized by those of skill in the art as predictive of *in vivo* effects at a catheter surface.

The MRD is constructed of an acrylic block, 42 cm long, with a lumen of 2x10 mm. The MRD is made of twenty-five evenly spaced specimen plugs each connected to a catheter latex segment whose anterior surface (0.3 cm²) comes in contact with the flushed infusate coming from a connected tubing and infusion bag. Several studies were conducted using this model, which are outlined in the following examples.

### EXAMPLE 3 - INHIBITION OF S. EPIDERMIDIS

The present example is provided to demonstrate the utility of the present invention for inhibiting *S. epidermidis* in and on a medical device, such as a catheter. The model described in Example 2 was used in the present study. These results demonstrate the utility of the invention for treating and maintaining catheter patency *in vivo*, and more specifically for inhibiting *S.* *epidermidis* adhesion and glycocalyx formation at a catheter surface.

Catheter segments were placed in the specimen plugs of the Modified Robbins Device described in Example 2. After placing the catheter segments in the specimen plugs, the entire apparatus was sterilized with ethylene oxide. A 500 ml 10% dextrose/water bag was infected with 4x10⁸ CFU/ml of *S. epidermidis* (to produce 8x10⁵ CFU per ml of D₁₀/W). The infected infusate was flushed through the MRD for 3 hours at a 50 ml/hr (using a peristaltic pump). In order to remove all free floating and loosely adherent staphylococci, the infected bag was removed and a new sterile bag (of D₁₀/W) was used to flush the MRD. The MRD was flushed with sterile D₁₀/W for 24 hours at 40 mls/hr. Following this, catheter segments of equal size were treated with different agents by placing them in tubes containing one of the following solutions:
1. Urokinase (5000 units/ml);
2. Heparin (1000 unit/ml);
3. EDTA (50 mg/ml); and
4. Trypsin (20,000 units/ml).

Representative catheter segments were then removed (in a sterile manner) at 4 and 24 hours and quantitatively cultured using the scrape-sonication technique described by Khoury *et al*. (1991)¹⁴ to isolate organisms adherent to catheter surfaces. The Khoury *et al*. reference is specifically incorporated herein by reference for this purpose. The experiment was done at 37°C.

The results from this study are presented at Table 2. The results demonstrate that treatment of catheter surfaces with EDTA was effective in preventing adherent *S. epidermidis* colonies on a catheter surface after only 4 hours of treatment. In contrast, urokinase, heparin and trypsin treatment of the catheter segments was significantly less effective at inhibiting adherent *S. epidermidis* colony formation and adherence after 4 hours of treatment.

**TABLE 2**

| **NO. OF ADHERENT *S. EPIDERMIDIS* COLONIES OBTAINED FROM 0.3 CM**^{**2**} **CATHETER SURFACES** | | |
|---|---|---|
| Agent Used | After 4 hrs. of treatment | After 24 hrs. of treatment |
| Urokinase | 310 | 40 |
| Heparin | 545 | 20 |
| EDTA | 0 | 0 |
| Trypsin | 150 | 5 |

### EXAMPLE 4 - M-EDTA AND THE PREVENTION OF BIOFILM FORMATION

The present example is provided to demonstrate the utility of the M-EDTA preparation in preventing glycocalyx-rich biofilm formation at the surface of a medical device, as well as to demonstrate the anti-staphylococcal activity of minocycline and EDTA preparations.

The method of Example 3 was used with the following modifications:
1. A more intense exposure to staphylococci (*S*. *epidermidis* and *S. aureus)* was achieved by flushing the MRD for 6 hours (instead of 3 hours in Example 3) with 3x10⁶ CFU of staphylococci/ml of D₅/W; and
2. The growth of adherent staphylococci to the catheter segments was promoted and achieved by exposing the catheter segments at 37°C to a 10% broth solution (prepared by adding 1 ml of trypticase soy broth to 9 ml of sterile H₂O) of EDTA (30 mg/ml of 10% broth solution), heparin (100 units/ml of 10% broth), urokinase (5,000 units/ml of 10% broth), minocycline (3 mg/ml of 10% broth), mino/EDTA (3 mg/30 mg per ml of 10% broth), vancomycin (3 mg/ml of 10% broth), vancomycin/heparin (3 mg vancomycin plus 100 units heparin/ml of 10% broth), or D₅/10% broth (50 mg/ml of 10% broth solution).

The results from these studies are demonstrated at Table 3 (*S*. *epidermidis*) and Table 4 (*S. aureus*).

**TABLE 3**

| **NO. OF ADHERENT *S. EPIDSRMIDIS* COLONIES OBTAINED FROM 0.3 CM**^{**2**} **CATHETER SURFACES** | | |
|---|---|---|
| Agent Used | After 4 hrs. of treatment | After 24 hrs. of treatment |
| Urokinase | >5x10³ | >5x10³ |
| Heparin | >5x10³ | >5x10³ |
| EDTA | 800 | 20 |
| Minocycline | 10 | 0 |
| Minocycline/EDTA | 0 | 0 |
| Vancomycin | 55 | 85 |
| Vancomycin/Heparin | 445 | 40 |
| D₅/10% broth | >5x10³ | >5x10³ |

As demonstrated in Table 3, the urokinase, heparin and dextrose solutions alone were equally ineffective in preventing and eradicating *S*. *epidermidis* adherence after 4 or 24 hours of catheter treatment. The minocycline and minocycline/EDTA provided effective prevention and irradication of *S. epidermidis* adhesion after only 4 hours of treatment. Minocycline/EDTA was slightly more effective than minocycline alone at 4 hours. EDTA alone and vancomycin/heparin provided minimal prevention at 4 hours but were more effective after 24 hours. Vancomycin alone provided equal partial prevention at 4 and 24 hours. M-EDTA was superior to all preparations examined, including vancomycin, vancomycin/heparin, minocycline or EDTA alone.

**TABLE 4**

| **NO. OF ADHERENT *S. AUREUS* COLONIES OBTAINED FROM 0.3 CM**^{**2**} **CATHETER SURFACES** | | |
|---|---|---|
| Agent Used | After 4 hrs. of treatment | After 24 hrs. of treatment |
| Urokinase | >5x10³ | >5x10³ |
| Heparin | 256 | >5x10³ |
| EDTA | 750 | 30 |
| Minocycline | 0 | 0 |
| Minocycline/EDTA | 0 | 0 |
| Vancomycin | 605 | 230 |
| Vancomycin/Heparin | 140 | 185 |
| D₅/10% broth | >5x10³ | >5x10³ |
| * All staphylococcus isolates were bloodstream slime-producing isolates obtained from human patient cases with catheter-related bacteremia. | | |

Table 4 demonstrates that minocycline and minocycline/EDTA solutions were the most effective inhibitors of *S. aureus* adhesion, with 0 adherent colonies being observed after 4 hours of treatment. EDTA alone, vancomycin alone and vancomycin/heparin were significantly less effective for preventing adherent *S*. *aureus*. These later three preparations had some partial anti-adherent activity, particularly after 24 hours of treatment.

These data (Table 3 and Table 4) demonstrate that minocycline alone or in combination with EDTA was effective for inhibiting *S*. *epidermidis* and *S*. *aureus* adherence and colonization of a catheter surface.

### EXAMPLE 5 - M-EDTA AND THE INHIBITION OF C. albicans ADHESION

The present example is provided to demonstrate the utility of the M-EDTA formulation in the inhibition of other glycocalyx and biofilm-forming microorganisms, such as *C*. *albicans*.

The M-EDTA flush formulation described in Example 1 was employed in the present example. The method employed was the same as that described at Example 4, with the following modifications. The organism used was a *C*. *albicans* obtained from the bloodstream of a patient with catheter-related candidemia. The infected infusate consisted of D₅/W with 4x10² CFU of *C. albicans* per ml flushed through the MRD for 6 hours. Results from the study are presented in Table 5.

**TABLE 5**

| **NO. OF ADHERENT *C. ALBICANS* OBTAINED FROM 0.3 CM**^{**2**} **CATHETER SURFACES** | | |
|---|---|---|
| Agent Used | After 4 hrs. of treatment | After 24 hrs. of treatment |
| Urokinase | >5x10³ | >5x10³ |
| Heparin | >5x10³ | >5x10³ |
| EDTA | 1060 | 155 |
| Minocycline | 190 | 535 |
| Minocycline/EDTA | 0 | 0 |
| D₅/10% broth | >5x10³ | >5x10³ |
| Vancomycin/heparin | >5x10³ | >5x10³ |
| Vancomycin | 470 | >5x10³ |

This example demonstrates minocycline/EDTA as a unique antistaphylococcal and antifungal agent. Vancomycin (a standard antistaphylococcal agent) when used alone or with heparin failed to have any anti-*C*. *albicans* activity and was not different from dextrose, urokinase or heparin solutions against *C. albicans.*

EDTA alone had some anti-*C*. *albicans* activity after 24 hours and minocycline alone had some activity at 4 and probably 24 hours. The combination of minocycline and EDTA (M-EDTA) provided a synergistic enhancement of anti-*C. albicans* activity, demonstrating an essentially total inhibitory effect against fungal adherence after 4 and 24 hours exposure. Therefore, M-EDTA is unique in preventing staphylococcal and Candida adherence to catheter surfaces (Staphylococci and Candida contributing to 95% to 100% of the pathogenic microbiology of catheter-related infections).

These results demonstrate that the solutions of a mixture of minocycline and EDTA provide a more effective and rapidly-acting preparation for the prevention of *S. epidermidis, S. aureus,* and *C. albicans* adhesion to a catheter surface than any other thrombolytic (urokinase), anticoagulant (heparin, EDTA), or antistaphylococcal preparation (minocycline, vancomycin, vancomycin/heparin).

### EXAMPLE 6 - S. epidermidis BIOFILM FORMATION AND HEPARIN, UROKINASE AND DEXTRINASE TREATMENT

The present example is provided to examine the relative *S. epidermidis* glycocalyx biofilm-destroying activity of heparin, urokinase and dextranase as assessed by scanning electron microscopy of an *S. epidermidis*-colonized catheter surface.

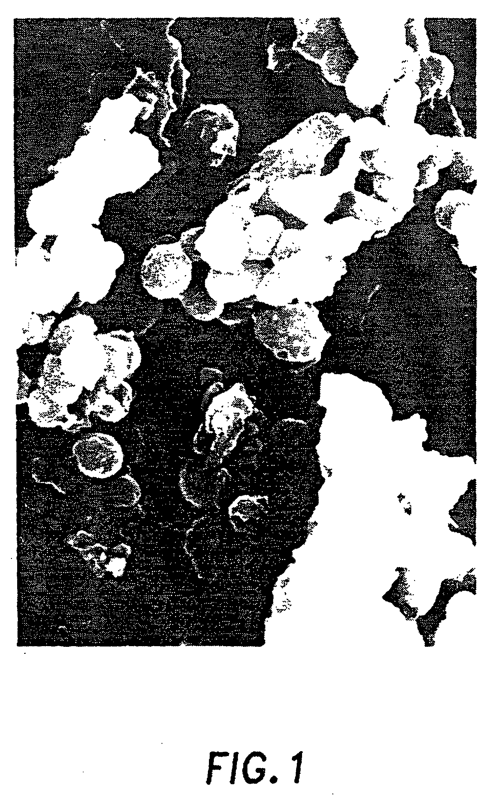

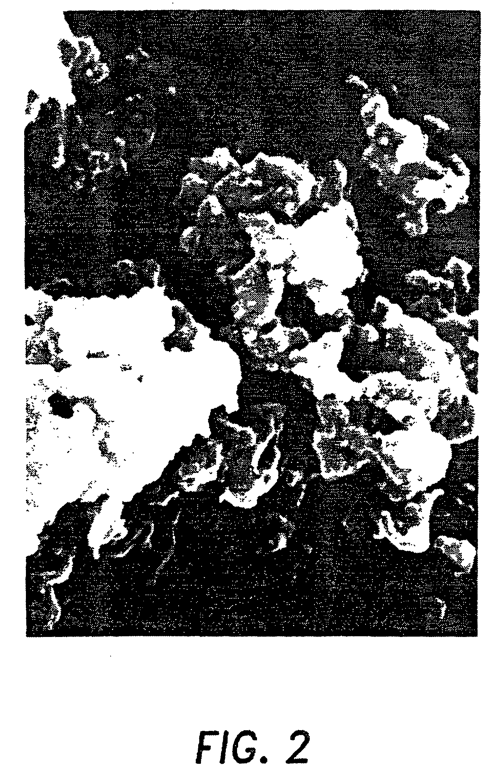

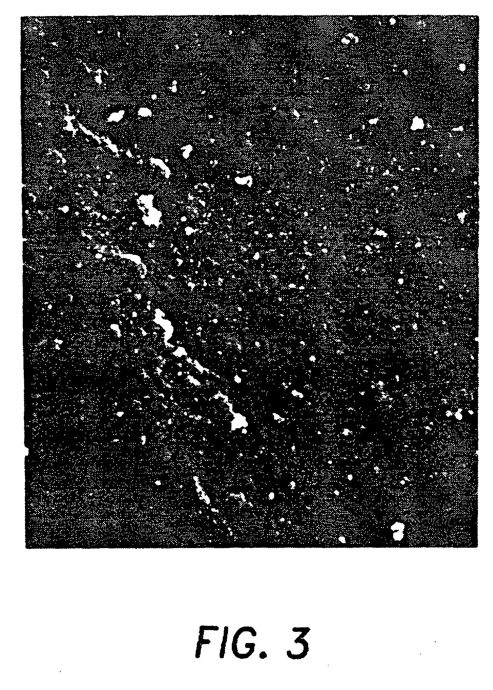

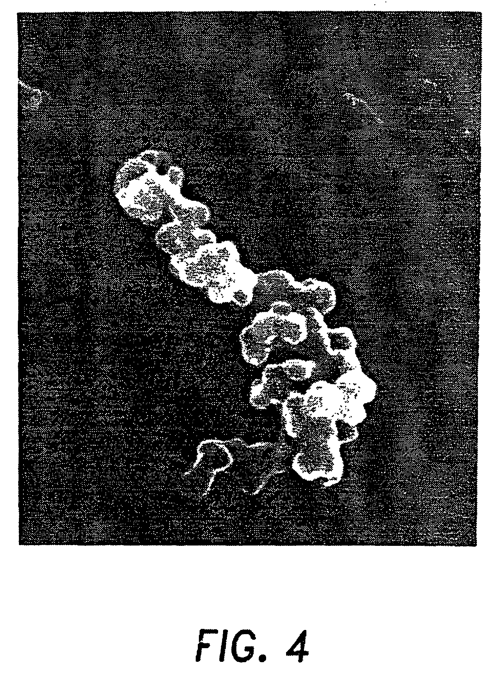

Scanning electron microscopy was done on various segments of a catheter exposed to *S*. *epidermidis* and then later exposed to heparin, urokinase or dextranase for 24 hours. A reduction in biofilm (glycocalyx) was noted on colonized catheter surfaces exposed to EDTA for 24 hours, compared to colonized surfaces later exposed to heparin, urokinase, or dextranase for 24 hours (Figure 1 = Dextranase; Figure 2 = Urokinase; Figure 3 = EDTA; Figure 4 = Saline).

### EXAMPLE 7 - PRETREATMENT OF CATHETER SURFACES WITH EDTA, Dextranase OR SALINE AND S. epidermidis BIOFILM FORMATION

The present example is provided to demonstrate the effect of chemically pretreating a catheter surface with EDTA or dextranase, compared to a saline control, on the formation of polysaccharide-rich biofilm formation, such as that characteristic of *S. epidermidis,* and the adhesion of these organisms to a catheter surface.

Catheter surfaces were coated with EDTA, dextranase or control (saline), at the concentrations described in Example 5, and then exposed to slime producing *S. epidermidis*.

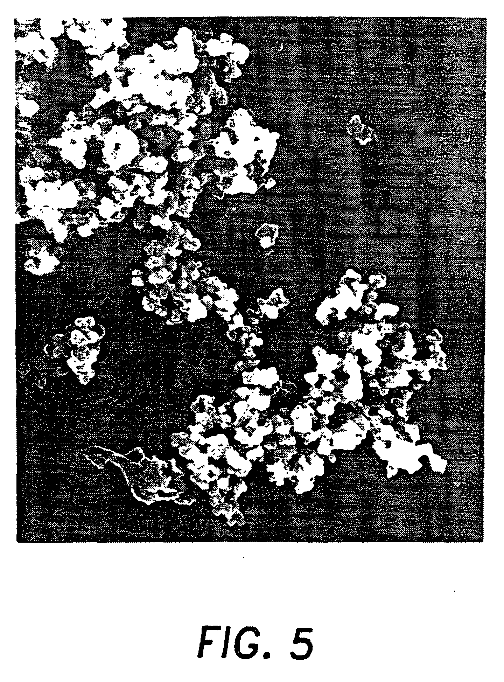

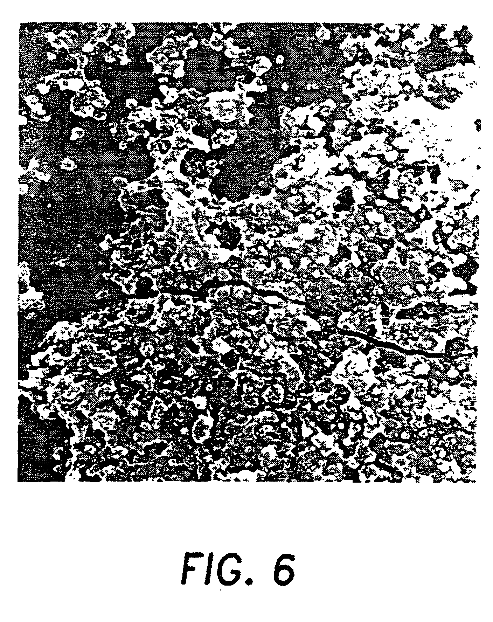

No biofilm or organisms were observed on EDTA coated surfaces. However, biofilm formation was observed on catheter surfaces pretreated with dextranase (Figure 4 and 5 = saline; Figure 3 = EDTA; Figure 6 = Dextranase pretreated).

### EXAMPLE 8 - MINOCYCLINE COATING OF A CATHETER AND MICROBIAL COLONIZATION

The present example is provided to demonstrate the anti-microbial colonization effect of the use of minocycline at a catheter surface.

Catheter surfaces were coated with minocycline, vancomycin or control cement with H₂O. Catheter surfaces were then exposed to clinical staphylococci isolates. The Modified Robbin's Device was employed in this study (See Example 2. The Modified Robbin's Device simulates a vascular catheter, and therefore provides a model predictive of *in vivo* effects.

One gram of methylmethacrylate (cement) was mixed with 0.5 ml of sterile H₂O and one of the following:
1. 60 mg of minocycline
2. 60 mg of vancomycin
3. control (cement + H₂O alone)

Equal amounts of cement alone or with minocycline or vancomycin were put in the lumen of catheter latex segments in a specimen plug of the Modified Robbin's Device. Twenty-four hours later, a one-liter infusate bag made of 5% dextrose in water was infected with 5 ml of 10⁵ to 10⁸ colony forming units (CFU) per ml of slime producing *Staphylococcus epidermidis* strains obtained from the bloodstream of patients with catheter related bacteremia. Using a peristaltic pump, the infected infusate was run for 2 hours at a rate of 60 ml/hr through the catheter segments of the Modified Robbin's Device.

Each catheter segment was made of 30 mm² silicone with a lumen filled with cement. At the end of 2 hours, some catheter segments (control and antibiotics coated) were taken out from specimen plugs and the cement in the lumen was removed, then the surface that was exposed to the infected fluid was cultured semiquantitatively using the roll-plate technique. Other segments were left behind and flushed with saline solution for 4 hours, then cultured by roll-plate.

Electron microscopy was used to document the adherence of staphylococci and the formation of biofilm layer on the surface of control uncoated catheter segments. Leaching of antibiotics from the cement was demonstrated to occur for at least one week by determining the inhibition around disc-shaped pieces of cement placed on blood agar plates that had been inoculated with bacteria. Coating of the catheter segments with antibiotics was demonstrated by the zone of inhibition that continued to form for at least one week around the disc-shaped catheter segments (without cement) placed on agar plates that had been inoculated with bacteria. The results from this study are presented in Table 6.

**TABLE 6**

| **NO. COLONIES OF S. EPIDERMIDIS FROM 30 MM**^{**2**} **CATHETER SURFACE** | | |
|---|---|---|
| Coating | Before Flush | After Flush |
| Control | 336 | 128 |
| Vancomycin | 174 | 111 |
| Minocycline | 48 | 15 |

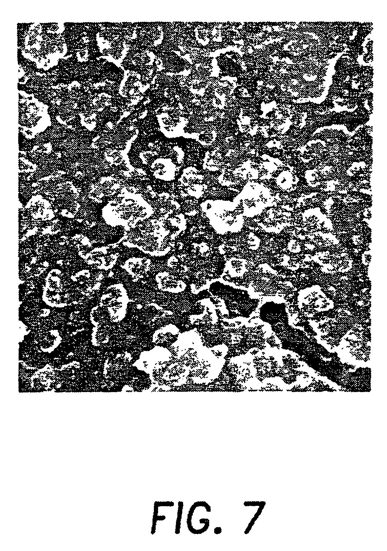

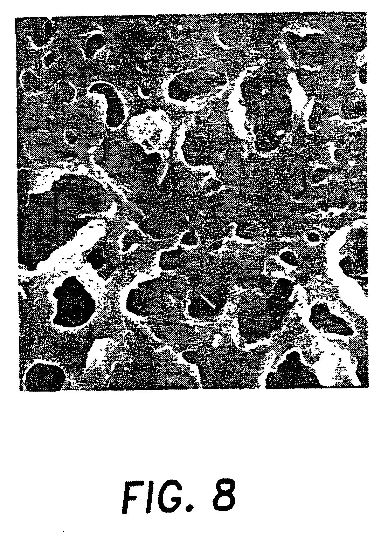

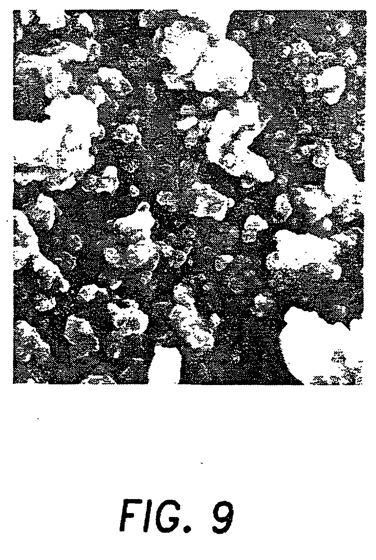

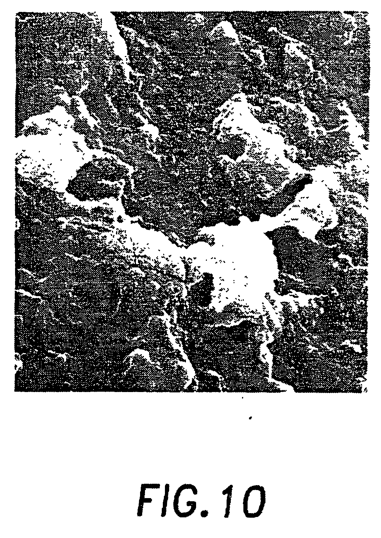

Catheter segments coated with minocycline had a significantly lower number of adherent *Staphylococcus epidermidis* colonies, compared to control and vancomycin coated catheter segments (see Table 6). However, fibrous glycocalyx was not inhibited on the minocycline-coated catheter surfaces. The scanning electron microscopic figures also evidenced these findings. See Figure 7 - shows fibrous glycocalyx on the surface of a control catheter segment - before flush; Figure 8 - shows some deranged fibrous glycocalyx on the surface of minocycline coated catheter segment - before flush; Figure 9 - shows fibrous glycocalyx on the surface of another control (saline) catheter segment after flush; and Figure 10 - shows fibrous glycocalyx on the surface of minocycline coated catheter segment after flush.

These data demonstrate that the coating of catheters with minocycline alone significantly reduced staphylococcal adherence. Fibrous glycocalyx formation is not inhibited with minocycline-coated surfaces.

### EXAMPLE 9 - STABILITY OF MINOCYCLINE AND EDTA PHARMACEUTICAL PREPARATIONS

The present studies will be conducted to characterize the stability of the pharmaceutical M-EDTA flushing solutions of the present invention. The M-EDTA solutions are expected to retain their potency for relatively long periods of time when stored refrigerated at about 4°C, i.e., for at least 1 month.

The solutions of the present invention have been examined for retained potency at room temperature (37°C). The M-EDTA solutions have been observed to retain relatively full anti-microbial and anti-fungal potency for at least 72 hours at room temperature. It is therefore expected that the formulation has a shelf life that renders it suitable for routine hospital use (Table 1 and Figure 11).

### EXAMPLE 10

### COMPARATIVE CLINICAL TRIAL OF M-EDTA AND HEPARIN FOR THE PREVENTION OF CATHETER-RELATED INFECTIONS

The present example outlines a study wherein the relative effectiveness of an M-EDTA catheter flushing solution will be compared to a heparin flushing solution (a currently used standard preparation) for the prevention of catheter-related infections and occlusions in humans.

The objective of these studies is to further document the utility of the M-EDTA flush solution as compared to a heparin flush solution in preventing infection and/or occlusion in central venous catheters (CVC).

Eligibility for enrollment into these studies will be based on the following inclusion/exclusion criteria:
**Inclusion Criteria**
   1. Patients must have a new (≤7 days old) functioning central venous catheter, utilized for infusion of chemotherapy, blood products, or other intermittent infusions.
   2. Patients must be able to return to the outpatient clinic for evaluation in case of CVC occlusion or occurrence of fever.
   3. Patients must have life expectancy for the planned duration of the study and must have catheter in place for study duration (study duration for a single patient is 6 months).
   4. Catheters in the percutaneous/tunneled group will be limited to Hickman/Broviac.
**Exclusion Criteria**
   1. Patients with an occluded central venous catheter.
   2. Patients with any existing local or systemic catheter infection.
   3. Patients with triple lumen catheters.
   4. Patients with polyurethane or teflon catheters.
   5. Patients currently taking warfarin.
   6. Patients requiring previous catheter removal due to venous thrombosis.
   7. It must be expected that the dwell time of 4 hours will not interfere with routine treatment of the underlying disease.
   8. Patients with Groshong catheters.

**Treatment Plan:** Patients will be randomly assigned and in double blind manner to have their CVC flushed with either M-EDTA or heparin according to the following:
1. Tunneled CVC (Hickman/Broviac) will receive either
   (a) two mls of M-EDTA (containing 3 mg of minocycline and 30 mg EDTA/ml) q daily
   (b) two mls of Heparin (100U/ml) q daily.
2. Infusion ports will receive either
   (a) two mls of M-EDTA (3 mg minocycline and 30 mg EDTA/ml) q 3 weeks
   (b) two mls of Heparin (100U/ml) q 3 weeks

Endpoints and Treatment Evaluation: All patients will be followed up for 6 months and will be evaluated for 2 endpoints: catheter infection/colonization and occlusion. Catheter infection will include local CVC-related infection or systemic catheter-related septicemia. Catheter colonization will include positive quantitative catheter culture (flush technique) or positive quantitative blood culture through the CVC in the absence of a positive peripheral blood culture or clinical manifestations of sepsis (fever, chills or hypotension). Patients in the study who develop fever will be evaluated, and simultaneous quantitative blood cultures through CVC and peripheral vein conducted. Catheter occlusion will be categorized as complete or partial depending on whether one cannot withdraw blood, infuse fluids through the CVC, or both. This subgroup of infected catheters will be analyzed separately.

Statistical Considerations: Based on a recent surveillance study conducted by the inventors (see Table 7), the rate of CVC-related sepsis in pediatric oncology patients ranges from 15% - 20.5% (see Table 6). These same ranges will be employed in further establishing the clinical utility of the treatment in adult patients.

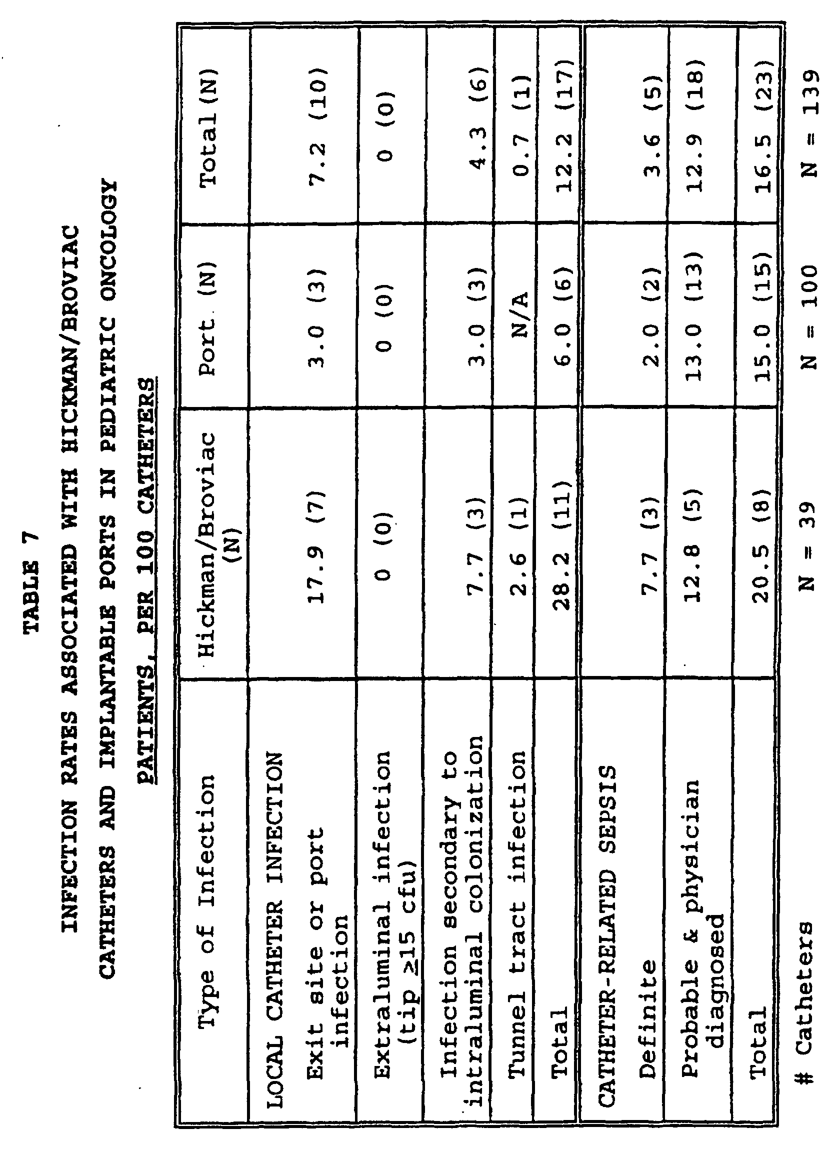

The results from this study will be employed in the development of a clinical protocol for the treatment and infection-free maintenance of indwelling catheters in humans.

### EXAMPLE 11 - PREPARATION OF M-EDTA-COATED DEVICES FOR IN VIVO USE

The present example is provided to demonstrate the utility of the M-EDTA solution as a coating material for medical devices, most particularly catheters.

Any of a variety of coating techniques may be used for imparting a protective covering of the M-EDTA solution to a device. By way of example, such methods include emersion of a medical device, such as a catheter, into a solution of minocycline and EDTA. This example provides a description of how these particular M-EDTA coated catheters were prepared.

Bioguard Cook Catheters with TDMACC surfactant were immersed in antibiotic solutions containing the following:
1. 60 mg of Minocycline plus 60 mg of EDTA/ml
2. 60 mg of Minocycline/ml
3. 60 mg of EDTA/ml

Catheters were immersed in each of the three solutions listed above for about 15 minutes. Bioguard Cook catheters not treated with any of the 3 solutions listed above (which are not coated by antimicrobials) were used as negative controls. Arrow Gard catheters coated with chlorhexidine and silver sulfadiazine were used as positive control devices. The Arrow Gard catheter is coated with antimicrobials. This catheter is described by Maki *et al*. in a clinical study, and reportedly decreased the rate of catheter-related bloodstream infection by five-fold as compared to a standard polyurethane triple-lumen CVC without a chlorhexidine and silver sulfadiazine coating.

### Other Coating Methods for Medical Devices

As noted, preparations of the present invention may be advantageously used as a coating preparation for treating the surfaces of a medical device. The medical devices which are amendable to coatings with the subject M-EDTA preparations generally have surfaces composed of thermoplastic or polymeric materials such as polyethylene, Dacron, nylon, polyesters, polytetrafluoroethylene, polyurethane, latex, silicone elastomers and the like. Devices with metallic surfaces are also amenable to coatings with the disclosed combinations. Such devices, for example indwelling catheters of types listed herein, can be coated by cement mixture containing the subject antibiotic compounds. Alternatively, devices that include a surfactant, such as the Bioguard Cook catheters, may be used whereby the surfactant at the surface of the device allows for the coating of the material onto the device. Particular devices especially suited for application of the M-EDTA preparation include intravascular, peritoneal, pleural and urological catheters; heart valves; cardiac pacemakers; vascular shunts; and orthopedic, intraocular, or penile prosthesis.

Various methods can be employed to coat the surfaces of medical devices with the M-EDTA coating preparation described herein (60 mg EDTA/60 mg minocycline). For example, one of the simplest methods would be to flush the surfaces of the device with the M-EDTA preparation. Generally, coating the surfaces by a simple flushing technique would require convenient access to the implantable device. For example, catheters, are generally amenable to flushing with a solution of EDTA and minocycline. For use as coating solutions, the effective concentration of minocycline would range from 0.001 to 100 mg/ml, preferably 60 mg/ml; and 1 to 100 mg/ml EDTA, preferably 60 mg/ml EDTA. The coating solution would normally be further composed of a sterile water or a sterile normal saline solutions.

A preferred method of coating the devices would be to first apply or adsorb to the surface of the medical device a layer of tridodecylmethyl ammonium chloride (TDMAC) surfactant followed by a coating layer of the M-EDTA preparation. This method for coating a device with M-EDTA provides for first absorbing to the catheter surface a cationic surfactant, such as TDMAC, to medical devices having a polymeric surface, such as polyethylene, silastic elastomers, polytetrafluoroethylene or Darcon, by soaking in a 5% by weight solution of TDMAC for 30 minutes at room temperature. The device should then be air dried, and rinsed in water to remove excess TDMAC. Alternatively, TDMAC pre-coated catheters are commercially available; for example, arterial catheters coated with TDMAC are available from Cook Critical Care, Bloomington, Indiana. The device carrying the adsorbed TDMAC surfactant coating can then be incubated in a solution of the M-EDTA combination for between about 15 minutes and an hour, washed in sterile water to remove unbound M-EDTA and stored in a sterile package until implantation. In general, the soaking solution of M-EDTA includes between 10 mg/ml - 100 mg/ml EDTA (preferably 60 mg/ml) and between 10 mg/ml - 100 mg/ml minocyclines (preferably 60 mg/ml) in an aqueous pH 7.4-7.6 buffered solution or sterile water.

Alternative processes and reagents for bonding an agent contained in a solution to a surfactant-coated implantable medical device are provided in U.S. Patent Nos. 4,442,133, 4,678,660 and 4,749,585. A further method useful to coat the surface of a medical device with an M-EDTA preparation involves first coating the selected surfaces with benzalkonium chloride followed by ionic bonding of the M-EDTA. *See, e.g.*, Solomon, D.D. and Sherertz, R.J. (1987)¹⁷ and U.S. Patent No. 4,442,133¹⁸.

Other methods of coating surfaces of medical devices with antibiotics are taught in U.S. Patent No. 4,895,566 (a medical device substrate carrying a negatively charged group having a pKa of less than 6 and a cationic antibiotic bound to the negatively charged group); U.S. Patent No. 4,917,686 (antibiotics are dissolved in a swelling agent which is adsorbed into the matrix of the surface material of the medical device); U.S. Patent No. 4,107,121 (constructing the medical device with ionogenic hydrogels, which thereafter absorb or ionically bind antibiotics); U.S. Patent No. 5,013,306 (laminating an antibiotic to a polymeric surface layer of a medical device); and U.S. Patent No. 4,952,419 (applying a film of silicone oil to the surface of an implant and then contacting the silicone film bearing surface with antibiotic powders).

These and many other methods of coating a solution to a solid surface appear in numerous patents and medical journal articles. As is evident, one of ordinary skill having benefit of this disclosure would be apprised of several different methods of coating a medical device surface with the subject inventive minocycline and EDTA coatings.

Medical devices, particularly catheters of the type listed in Table 8, may be coated with the M-EDTA solution and then stored in a sterile packaging material until use.

**TABLE 8**

| SHORT-TERM TEMPORARY ACCESS CATHETER | LONG-TERM INDEFINITE VASCULAR ACCESS |
|---|---|
| Peripheral intravenous cannulas | Peripherally inserted central venous catheters (PICC) |
| - winged steel needles | |
| - peripheral intravenous catheters | |
| Arterial catheters | Percutaneous nontunneled silicone catheters |
| Central venous catheters | Cuffed tunneled central venous catheters (Hickman and Broviac) |
| Swan-Ganz catheters | Subcutaneous central venous ports (Infusaport, Port-a-cath, Landmark) |
| Hemodialysis catheters | |
| Umbilical catheters | |

### EXAMPLE 12

### METHOD FOR MAINTAINING CATHETER PATENCY WITH MINOCYCLINE-EDTA PHARMACEUTICAL PREPARATION

The present example demonstrates one embodiment of a method that may be used in maintaining the patency of an indwelling catheter in a patient. The regimen described herein is potentially applicable for use in adult patients, as the dose of M-EDTA in the regimen exposes patients only to relatively low, pharmaceutically acceptable levels of the EDTA and minocycline, and has already been demonstrated by the inventors as effective in pediatric patients.

An indwelling catheter of a patient will be flushed with a solution of minocycline/EDTA. The "flushing" of the catheter will constitute filling the catheter with a volume of the M-EDTA solution sufficient to provide a concentration of 9.0 mg minocycline and a concentration of between 90 mg EDTA in the catheter. For example, assuming a catheter volume of 2-3 ml., the solution will contain a concentration of EDTA of between 10 mg/ml - 30 mg/ml, and a concentration of minocycline of between 1-3 mg/ml. "Flushing" the catheter with 3 ml of the M-EDTA solution will thereby provide a dose of between 3-9 mg minocycline and 30 - 90 mg EDTA. The solution of M-EDTA will be prepared as outlined in Example 1.

The "flushing" of the catheter is achieved by adding between 2-3 ml of the M-EDTA solution to the catheter. The solution is then allowed to diffuse through the catheter to the patient in which it is implanted. The concentration of the EDTA and minocycline in the solution is such that the patient will be exposed only to concentrations of the agents well below pharmacologically tolerable levels.

The flushing of the catheter is to be repeated at periodic intervals of at least between every 24 to 72 hours (preferably, every 24 hours) to assure that infectious organisms are not allowed an opportunity to colonize the surface or initiate biofilm formation on the catheter surface.

### EXAMPLE 13

### EFFICACY OF ANTIBIOTIC COATED CATHETERS AFTER GAS STERILIZATION

The present example is provided to demonstrate the stability of the coated devices to sterilization processes. In order to test the effect of gas sterilization on catheters coated with Minocycline, EDTA and the combination of drugs, the following studies were performed.

M-EDTA-coated catheters were prepared as described in Example 11. The catheters were then divided into three representative groups. The catheter antibiotic activity was determined in vitro by a modified Kirby-Bauer technique described in Sherertz *et al*. ((1989) *Antimicrob. Agents Chemother.,* 33:1174-1178). The first set of catheters were tested immediately after immersion without gas sterilization. The second set was tested 24 hours later without gas sterilization. The third set was tested 24 hours after gas sterilization.

The modified Kirby-Bauer technique consisted of growing a strain of slime producing catheter-related bacteremic isolate of *Staphylococcus epidermidis* for 18 hours in trypticase soy broth, then diluting the solution to 10 CFU ml in phosphate-buffered saline. A cotton swab was placed in the staphylococcal suspension and then rubbed across the surface of a trypticase soy agar plate. Individual catheters were cut into 20 mm lengths pressed into agar overlaid with *S*. *epidermidis* and incubated overnight at 37°C. Zone sizes were assessed by measuring the diameter perpendicular to the long axis of the catheter. The data in Table 9 demonstrates that Catheters treated with the M-EDTA preparations maintained the greatest post-sterilization zone of inhibition (0 hours =40; 24 hours = 34).

For the *in vivo* studies, the catheters were cut into 2 cm segments and then emersed in 60 mg/ml EDTA, 60 mg/ml minocycline solution each and then immersed in a solution of a mixture containing 60 mg/ml mino and 60 mg/ml EDTA for 15 minutes. All catheters were allowed to dry for one hour and they were gas sterilized.

Table 9 provides the results achieved with these studies. This data demonstrates the superior anti-*S*. *epidermis* activity of the M-EDTA coating as compared to non-treated as well as the Arrow Gard catheters.

**TABLE 9**

| ***S. EPIDERMIDIS* ZONE OF INHIBITION - DIAMETER (mm)** | | | |
|---|---|---|---|
| | Pre Sterilization | | Post Sterilization |
| | 0 Hrs | 24 Hrs | 24 Hrs |
| Minocycline | 36 | 33 | 18 |
| M-EDTA | 40 | 34 | 35 |
| EDTA | 5 | 18 | 10 |
| Control | 0 | 0 | 0 |
| Arrow Gard* | 13 | 15 | 7 |

| | | | |
|---|---|---|---|
| *Coated with chlorhexidine gluconate and silver sulfadiazine | | | |

### EXAMPLE 14

### EFFICACY OF ANTIBIOTIC COATED CATHETERS AFTER GAS STERILIZATION TO S. AUREUS

The present example is provided to further demonstrate the stability of the described M-EDTA coatings to sterilization processes, such as gas sterilization, particularly as measured through the retained anti-microbial activity of the device.

The protocol of Example 13 was used to prepare the catheters used in this study (using a catheter-related bacteremic strain of *S*. *aureus).* The 0 hour catheters, and catheters coated with minocycline or EDTA alone were not included in the study.

**TABLE 10**

| ***S*. *AUREUS* ZONE OF INHIBITION - DIAMETER (mm)** | | |
|---|---|---|
| | Pre Sterilization | Post Sterilization |
| | 24 Hrs | 24 Hrs |
| M-EDTA | 31 | 29 |
| Control | 0 | 0 |
| Arrow Gard* | 13 | 13 |

| | | |
|---|---|---|
| *Coated with chlorhexidine gluconate and silver sulfadiazine | | |

The present example demonstrates that gas sterilization did not affect the antibiotic activity of minocycline-EDTA coated catheters and that these catheters were at least two times more active at inhibiting *Staphylococcus aureus* than the Arrow septic catheters.

### EXAMPLE 15

### COMPARATIVE EFFICACY OF M-EDTA COATED CATHETERS AGAINST CATHETER RELATED MICROORGANISMS

The present example demonstrates the broad spectrum activity of M-EDTA catheters. The same zone of inhibition measure described in examples 13 and 14 (with the modified Kirby-Bauer technique) were employed using M-EDTA coated catheters and Arrow Gard catheters (commercially available) to examine the effectiveness of the various coatings against different catheter-related organisms such as *Staphylococcus epidermidis, Staphylococcus aureus, Candida albicans* and gram negative bacilli (*Pseudomonas aeruginosa, Xanthomonas maltophilia,* and acinetobacter species.) Approximately 60% of catheter infections are caused by *S. epidermidis*, 10% by *S. aureus,* 10% by *C. albicans,* 20% by gram negative bacilli (mostly *P. aeruginosa, X. maltophilia,* and acinetobacter species.) These studies show that catheters coated with M-EDTA have a broad spectrum activity against different species of bacteria and fungi as well as different strains of the same species. For purposes of the present studies, a zone of inhibition of ≥ 15 mm is a predictor of excellent efficacy in veins. A zone of 10 - 15 mm is a predictor of moderate efficacy and a zone of inhibition of ≤ 10 mm is a predictor of poor efficacy.

### Staphylococcus epidermis

The data in Table 11 demonstrates that the M-EDTA coated catheters has a significantly greater zone of inhibition to five strains of staphylococcus epidermidis, as compared to non-M-EDTA coated catheters (Arrow Gard catheters).

**TABLE 11 -**

| **ZONES OF INHIBITION (mm) (STAPHYLOCOCCUS EPIDERMIDIS (SE) STRAINS** | | |
|---|---|---|
| Strain No. | Arrow Gard | Mino/EDTA |
| SE 4392 | 14 | 31 |
| SE 3996 | 16 | 29 |
| SE 4345 | 17 | 39 |
| SE 4023 | 15 | 29 |
| SE 93 | 15 | 31 |
| Mean (SD) | 15.4 (±1.1) | 31.8 (±4.14) |
| p = 0.001 | | |

### Staphylococcus aureus

Table 12 sets forth data obtained employing five different strains of *Staphylococcus aureus* (SA) in the aforedescribed zone of inhibition assays. The data demonstrate that the M-EDTA coated catheters provided a significantly greater zone of inhibition compared to the non-M-EDTA catheters (Arrow Gard).

**TABLE 12 -**

| **ZONES OF INHIBITION - DIAMETER (mm)** | | |
|---|---|---|
| Strain No. | Arrow Gard | Mino/EDTA |
| SA 1445 | 13 | 23 |
| SA 1432 | 15 | 28 |
| SA 1414 | 12 | 23 |
| SA 48 | 14 | 23 |
| SA 1411 | 12 | 34 |
| Mean (SD) | 13.2 (±1.3) | 26.2 (±4.9) |
| p = 0.005 | | |

### Candida albicans

Table 13 sets forth data obtained employing the organism *Candida albicans*, in the zone of inhibition study protocol, comparing the inhibitory action of the M-EDTA and non-M-EDTA coated catheters especially. "Ampho B" stands for a broad spectrum antibiotic. The data in Table 13 demonstrates that the M-EDTA treated catheters had superior anti-*Candida albicans* inhibitory activity as compared to control and the three other catheter types (coatings) tested.

**TABLE 13 -**

| **ZONES OF INHIBITION - DIAMETER (mm)*Candida albicans*** | | | |
|---|---|---|---|
| | Trial #1 | Trial #2 | Trial #3 |
| M-EDTA | 16 | 21 | 16 |
| Minocycline | 0 | 0 | 0 |
| Control | 0 | 0 | 0 |
| Arrow Gard | 10 | 9 | 9 |
| Ampho B | ND | 18 | 18 |

Table 14 sets forth data obtained in studies with five strains of *Candida albicans*, and again demonstrates the broad range anti-microbial activity of the M-EDTA coated catheters.

**TABLE 14 -**

| **ZONES OF INHIBITION - DIAMETER (mm) (STRAINS OF *CANDIDA ALBICANS* (CA)** | | |
|---|---|---|
| Strain No. | Arrow Gard | Mino/EDTA |
| CA 291 | 0 | 16 |
| CA 596 | 10 | 12 |
| CA 276 | 10 | 10 |
| CA 267 | 10 | 13 |
| CA 319 | 7 | 18 |
| Mean (SD) | 7.4 (±4.3) | 13.8(±3.2) |
| p = 0.030 | | |

### Acinetobacter (Acin)

Table 15 demonstrates the efficacy of the described M-EDTA coatings for inhibiting Acinetobacter.

**TABLE 15 -**

| **ZONES OF INHIBITION - DIAMETER (mm)** | | | | |
|---|---|---|---|---|
| Strain No. | M/EDTA | TMP/SMX | CFTZ | ARROW GARD |
| ACIN639 | 30 | 00 | 00 | 00 |
| ACIN38B | 23 | 30 | 15 | 05 |
| ACIN632 | 24 | 10 | 00 | 00 |
| ACIN633 | 10 | 15 | 00 | 09 |
| ACIN1771 | 43 | 14 | 00 | 12 |
| Mean* | 26 | 14 | 3.0 | 5.2 |
| STDEV | 12 | 11 | 6.7 | 5.3 |
| M = Minocycline; CFTZ = Ceftazidime; | | | | |
| TMP-SMX = Trimethoprin-Sulfamethoxazole | | | | |

| | | | | |
|---|---|---|---|---|
| * The efficacy of the catheter coated with M-EDTA was significantly higher than the Arrow Gard (p = 0.016) | | | | |

### P. Aeruginosa (PSA) Strains

Table 16 demonstrates the efficacy of the M-EDTA coated catheters against *P. aeruginosa* (PSA) strains.

**TABLE 16 -**

| **ZONES OF INHIBITION - DIAMETER (mm)** | | | | |
|---|---|---|---|---|
| Strain No. | M/EDTA | TMP/SMX | CFTZ | ARROW GARD |
| PSA1644 | 15 | 00 | 13 | 03 |
| PSA2455 | 11 | 05 | 20 | 00 |
| PSA2451 | 11 | 00 | 20 | 06 |
| PSA2456 | 14 | 00 | 29 | 00 |
| PSA2452 | 10 | 00 | 28 | 06 |
| Mean* | 12.2 | 1.0 | 22 | 3.0 |
| STDEV | 2.17 | 2.24 | 6.60 | 3.0 |

| | | | | |
|---|---|---|---|---|
| * The efficacy of the catheter coated with M-EDTA was significantly higher than the Arrow Gard (p = 0.009) | | | | |

### X. Maltophilia (XMAL) Strains

Table 17 demonstrates the efficacy of the M-EDTA catheters against *X. maltophilia* strains.

**TABLE 17 -**

| **ZONES OF INHIBITION - DIAMETER (mm)** | | | | |
|---|---|---|---|---|
| Strain No. | M/EDTA | TMP/SMX | CFTZ | ARROW CARD |
| XMAL5653 | 24 | 37 | 32 | 12 |
| XMAL2496 | 34 | 22 | 29 | 00 |
| XMAL8929 | 37 | 40 | 20 | 00 |
| XMAL2657 | 35 | 30 | 15 | 04 |
| XMAL2172 | 15 | 15 | 20 | 00 |
| Mean* | 29 | 28.8 | 23.3 | 3.0 |
| STDEV | 9.3 | 10.38 | 7.05 | 5.22 |

| | | | | |
|---|---|---|---|---|
| * The efficacy of the catheter coated with M-EDTA was significantly higher than the Arrow Gard (p = 0.0016) | | | | |

The aforegoing results demonstrate that M-EDTA coated catheters have a broad spectrum activity against various microbial agents that can cause CVC related infections. This activity in the M-EDTA coated catheters is superior and provides a broader spectrum anti-microbial activity than that provided using Arrow Gard catheters.

### EXAMPLE 16

### SHELF LIFE AND STABILITY OF THE COATED M-EDTA (IN SERUM)

Catheters coated with M-EDTA, as well as control and Arrow catheters were tested at baseline (day 1 - D1) against *S*. *epidermidis* using a zone of inhibition measure for relative anti-microbial activity. Then the same catheters were placed in serum at 37°C and tested at days 3, 7, 15 and 30 to determine inhibitory efficacy over time. (Table 17). In addition, segments of the same catheters were kept for 30 and 60 days at 25°C then tested to determine anti-microbial activity as a measure of the shelf life of cetheters with such coatings (Table 18).

### STABILITY OF M-EDTA COATED CATHETERS

**TABLE 17 -**

| **ZONES OF INHIBITION - DIAMETER (mm)** | | | | | |
|---|---|---|---|---|---|
| | D1 | D3 | D7 | D15 | D30 |
| M-EDTA | 31 | 21 | 16 | 14 | 10 |
| Control | 0 | 0 | 0 | 0 | 0 |
| Arrow Gard | 14 | 07 | 07 | 05 | 03 |

**TABLE 18 -**

| **ZONES OF INHIBITION - DIAMETER (mm)** | | | |
|---|---|---|---|
| | DAY 30 | | DAY 60 |
| | Serum 37°C | 25°C | 25°C |
| M-EDTA | 10 | 34 | 32 |
| Control | 0 | 0 | 0 |
| Arrow Gard | 3 | 13 | 12 |

The foregoing studies demonstrate that M-EDTA coated catheters maintain excellent anti-microbial (particularly anti-S epidermidis) efficacy for at least two weeks in serum at 37°C and at least two months at 25°C. Whereas, the efficacy of the Arrow Gard catheters decreases rapidly within three days in serum (37°C).

### EXAMPLE 17

### IN VIVO EFFICACY OF M-EDTA COATED CATHETERS IN A RABBIT MODEL

Animal studies with polyurethane catheters coated with minocycline plus EDTA are provided. The results are consistent with the extensive *in vitro* data described herein. Currently, the only antibiotic-coated catheters on the market are those produced by Arrow. These catheters are coated with chlorhexidine gluconate and silver sulfadiazine. They have been described in a clinical study done by Maki *et al*. as capable of reducing the rate of catheter-related sepsis five fold.

An established *in vivo* rabbit model was used in the present example (Sherertz *et al*. *Journal of Infectious Diseases* 167:98-106, 1993). Catheters were inserted percutaneously in the rabbit under aseptic conditions. Immediately after insertion, the catheter insertion site was inoculated with 0.1 ml of 10⁵ colony forming units (CFU) of *Staphylococcus aureus* from the bloodstream of a patient with catheter-related *S. aureus* bacteremia. Seven days after insertion, the catheters were removed and the subcutaneous (SQ) as well as the tip of the catheters were cultured by quantitative catheter cultures.

### PREPARATION OF M-EDTA COATED CATHETERS

Catheters were first pretreated with a cationic surfactant tridodecylmeylammonium chloride (TDMAC). (Example 11). Other surfactants, such as benzalkonium chloride, may also be used. Treatment of a catheter with a surfactant will enable subsequent bonding of anionic substances, such as the antibiotic minocycline (mino) and EDTA to the surface. For the present studies, polyethlylene catheters already coated with TDMAC were immersed in a 60 mg/ml solution of EDTA and 60 mg/ml minocycline, (Bio-Guard AB coating, Cook Critical Care, Bloomington, Ind.). These polyethylene catheters were manufactured by Cook Critical Care (Bloomington, Ind.), 5.0 Fr (18 ga), 15 cm (5 7/8 in)). For these *in vivo* studies, the catheters were cut into 6 cm segments before treating with the M-EDTA solution.

Solution A. 3 vials of minocycline (100 mg each) diluted with 0.8 ml of sterile water for injection, USP to obtain 2.4 ml of 120 mg/ml minocycline;

Solution B. 1.6 ml of 150 mg/ml EDTA was added to 0.4 ml sterile water to obtain 2 ml of 120 mg/ml EDTA. Finally, 2 ml of solution A was added to 2 ml solution B, resulting in a 4 ml solution of 60 mg/ml minocycline and 60 mg/ml EDTA.

All catheters used were of the polyurethane type made by Arrow or Cook. The following results were obtain in the experiment where catheters were cultured by either the standard semiquantitative roll-plate culture technique (Maki *et al*., 1977, *N. Engl. J. Med.,* 296:1305-1309) or the sonication (Sherertz *et al*., 1990, *J. Clin. Microbiol*., 28:76-82) technique.

The data in Tables 19 and 20 demonstrated the consistently inhibitory activity of the M-EDTA coated catheters against S. aureus colonization.

**TABLE 19 -**

| **NUMBER OF *S*. *AUREUS* COLONIES CULTURED BY ROLL-PLATE** | | |
|---|---|---|
| Catheter Type | Catheter Tip | Catheter SO* |
| Control | >1000 | >1000 |
| Control | >1000 | >1000 |
| Arrow Gard^{†} | 2 | 10 |
| Arrow Gard | 0 | 0 |
| Arrow Gard | 8 | 10 |
| Arrow Gard | 2 | 8 |
| M-EDTA | 0 | 0 |
| M-EDTA | 0 | 0 |

| | | |
|---|---|---|
| † Coated with chlorhexidine gluconate and silver sulfadiazine | | |
| * Subcutaneous catheter segment | | |

**TABLE 20 -**

| **NUMBER OF *S*. *AUREUS* COLONIES CULTURED BY ROLL-PLATE** | | |
|---|---|---|
| Catheter Type | Catheter Tip | Catheter SO* |
| Control | >1000 | >1000 |
| Control | 18 | 25 |
| Arrow Gard† | >1000 | >1000 |
| Arrow Gard | 1 | 16 |
| M-EDTA | 0 | 0 |
| M-EDTA | 0 | 0 |

The data in Table 21 was obtained using catheter segments cultured by the quantitative sonication technique (as described by Sheretz *et al*. (1990). This data demonstrates again the conssitently anti-S. aureus colonizing effect provided through coating a catheter or other device with a combination of minocycline and EDTA.

**TABLE 21 -**

| **NUMBER OF *S*. *AUREUS* COLONIES CULTURED BY SONICATION** | | |
|---|---|---|
| Catheter Type | Catheter Tip | Catheter SO* |
| Control | 40 | 60 |
| Control | 380 | >1000 |
| Arrow Gard† | 180 | >1000 |
| Arrow Gard | 0 | 20 |
| M-EDTA | 0 | 0 |
| M-EDTA | 0 | 0 |

These studies demonstrate the complete prevention of colonization upon using M-EDTA coated catheters compared to the partial prevention achieved using the Arrow Gard catheters.

### EXAMPLE 18

### IN VIVO EFFICACY OF M-EDTA CATHETERS

The present example further demonstrates the anti-microbial activity of the coated M-EDTA devices *in vivo*. The M-EDTA-coated catheters were prepared as described in Example 17. 10⁴ colony forming units of *S. aureus* were inoculated at the insertion site of a catheter coated with M-EDTA (as described in Example 13), with chlorhexidine gluconate and silver sulfadiazine (the Arrow Gard catheter) or with TDMAC alone with no added antibiotic.

The rabbit model described in Sherertz *et al.* (1993) was used in the present study, i.e., New Zealand white rabbits between 2-3 months old and weighing 2-3 kg. The data in Table 22 demonstrates the superior antistaphylococcal and total inhibitory activity of MEDTA as compared to the partial *anti-S. aureus* activity achieved with the Arrow Gard catheter.

**TABLE 22-**

| **NUMBER OF *S. AUREUS* COLONIES CULTURED BY ROLL-PLATE** | | | | |
|---|---|---|---|---|
| Catheter No. | Catheter Type | Catheter Tip | Catheter SQ | Catheter Site Purulence |
| 1 | Control | > 1000 | > 1000 | Yes |
| 2 | Control | > 1000 | > 1000 | Yes |
| 3 | Control | > 1000 | > 1000 | Yes |
| 4 | Control | > 1000 | > 1000 | Yes |
| 5 | Arrow Gard | 3 | 10 | No |
| 6 | Arrow Gard | 15 | 10 | No |
| 7 | Arrow Gard | 15 | 15 | No |
| 8 | Arrow Gard | 20 | 15 | No |
| 9 | M-EDTA | 0 | 0 | No |
| 10 | M-EDTA | 0 | 0 | No |
| 11 | M-EDTA | 0 | 0 | No |
| 12 | M-EDTA | 0 | 0 | No |
| 13 | M-EDTA | 0 | 0 | No |
| 14 | M-EDTA | 0 | 0 | No |
| 15 | M-EDTA | 0 | 0 | No |
| 16 | M-EDTA | 0 | 0 | No |

### EXAMPLE 19 - IN VIVO COMPARATIVE STUDY OF M-EDTA-COATED CATHETERS V. CHLORHEXIDINE/SILVER SULFADIAZINE-COATED CATHETERS

For this example, catheters were cultured by a sonication quantitative culture technique described in Sheretz et al. (1990). The sonication of technique is a quantitative culture technique, and is described in Raad *et al*. (1992) (Diagn. Microbiol. Infect. Dis., 15:13-20). The M-EDTA catheters were coated as described in Example 11. The chlorhexidine/silver sulfadiazine coated catheters are commercially available from Arrow International, Inc. (300 Bernville Road, Reading, PA 19605) (Arrow Gold catheters). The data in Table 23 again demonstrates the consistently effective anti-microbial, particularly the anti-S. aureus, activity of the M-EDTA coated devices of the invention. As well as the superior anti-microbial activity of the M-EDTA devices as compared to the Arrow Gard catheter.

**TABLE 23 -**

| **NUMBER OF *S*. *AUREUS* COLONIES CULTURED BY SONICATION** | | |
|---|---|---|
| Catheter Type | Catheter Tip | Catheter SO |
| Control | 600 | 520 |
| Control | 400 | 480 |
| Control | 600 | N/A |
| Control | 640 | N/A |
| Arrow Gard | 40 | 80 |
| Arrow Gard | 120 | 80 |
| Arrow Gard | 0 | 160 |
| Arrow Gard | 80 | 160 |
| M-EDTA | 0 | 0 |
| M-EDTA | 0 | 0 |
| M-EDTA | 0 | 0 |
| M-EDTA | 0 | 0 |
| M-EDTA | 0 | 0 |
| M-EDTA | 0 | 0 |
| M-EDTA | 0 | 0 |
| M-EDTA | 0 | 0 |

### EXAMPLE 20 - IN VIVO ANTI-MICROBIAL ACTIVITY OF M-EDTA COATED CATHETERS

This study was performed in the rabbit model as described in Example 18. 10⁴ colony forming units (CFU) *of S. aureus* (a PI strain) were used to infect the catheter insertion site. The catheters were cultured by sonication. (Sheretz *et al.* (1990)). The Arrow Gard and Cook catheters coated with EDTA (Example 17) were employed in the study. The control catheters used were again the Cook catheters that have a coating of TIDMAC, but are without antibiotic or EDTA. Table 24 provides the date collected in this study. The results in Table 24 again demonstrate the *in vivo* effectiveness of the M-EDTA coated catheters for inhibiting infection by *S. aureus in vivo*.

Catheters were inserted into the subcutaneous space of New Zealand white rabbits 2-3 months old and weighing 2-3 kg. A 0.1 ml of 10 colonies of a virulent *S. aureus* strain (PI strain) was injected at the insertion site. The rabbits were sacrified on day 7. Catheters were aseptically removed and the 2 cm tip cultured by the sonication technique. The results are shown in Table 24. These results again demonstrate the *in vivo* effectiveness of M-EDTA against *S*. *aureus* infection. These results confirm previous experiments whereby M-EDTA coated catheters prevented staphylococcal colonization and catheter infection compared to partial prevention by the Arrow catheters.

**TABLE 24 -**

| **NUMBER OF *S*. *AUREUS* COLONIES CULTURED BY SONICATION** | | | |
|---|---|---|---|
| Catheter No. | Catheter Type | Catheter Tip | Catheter Site Purulence |
| 1 | Control | > 10⁵ | Yes |
| 2 | Control | > 10⁵ | Yes |
| 3 | Control | > 10⁵ | Yes |
| 4 | Control | > 10⁵ | Yes |
| 5 | Arrow Gard | > 10³ | No |
| 6 | Arrow Gard | 16 | No |
| 7 | Arrow Gard | 0 | No |
| 8 | Arrow Gard | 0 | No |
| 9 | Arrow Gard | 0 | No |
| 10 | Arrow Gard | 0 | No |
| 11 | Arrow Gard | 0 | No |
| 12 | Arrow Gard | 0 | No |
| 13 | Arrow Gard | 0 | No |
| 14 | M-EDTA | 0 | No |
| 15 | M-EDTA | 0 | No |
| 16 | M-EDTA | 0 | No |
| 17 | M-EDTA | 0 | No |
| 18 | M-EDTA | 0 | No |
| 19 | M-EDTA | 0 | No |
| 20 | M-EDTA | 0 | No |
| 21 | M-EDTA | 0 | No |
| 22 | M-EDTA | 0 | No |

### BIBLIOGRAPHY

1. Harper WE and Epis JA (1987), Effect of chlorhexidine/EDTA/Tris against bacterial isolates from clinical specimens., *Microbios*., *51*:107-112.
2. Said AA *et al*. (1987), Expression of H1 outer-membrane protein of *Pseudomonas* aeruginosa in relation to sensitivity to EDTA and polymyxin B., *J. Med. Microbiol*., *24*:267-274.
3. Root JL *et al*. (1988), Inhibitory effect of disodium EDTA upon the growth of *Staphylococcus epidermidis in vitro:* Relation to infection prophylaxis of Hickman catheters., *Antimicrob. Agents Chemother*., *32*:1627-1631.
4. Clumeck N *et al*. (1984), Treatment of severe staphylococcal infections with a rifampin-minocycline association., *J*. *Antimicrob*. *Chemother*., *13*(S):17-22.
5. Yourassowsky E *et al*. (1981), Combination of minocycline and rifampin against methicillin and gentamicin resistant *Staphylococcus aureus., J. Clin. Pathol., 34*:559-563.
6. Zinner SH *et al*. (1981), Antistaphylococcal activity of rifampin with other antibiotics., *J. Infect. Dis*., *144*365-371.
7. Segreti J *et al*. (1989), *In vitro* activity of minocycline and rifampin against staphylococci., *Diagn. Microbiol. Infect. Dis., 12*:253-255.
8. Yuk JH *et al*. (1991), Minocycline as an alternative antistaphylococcal agent., *Rev*. *Infect. Dis., 13*:1023-1024.
9. Root *et al*. (1988), Inhibitory Effect of Disodium EDTA upon the Growth of *Staphylococcus* epidermidis *In Vitro:* Relation to Infection Prophylaxis of Hickman Catheters., *Antimicrobial Agents and Chemotherapy, 32*(11):1627-1631.
10. Goodman and Gilman's *The Pharmacological Basis of Therapeutics,* 8th edition (1990), Pergamon Press
11. The Merck Index, 11th edition (1989), Merck & Co., Inc. Publishers, p. 1621.
12. Machnicka *et al*. (1986), *Folia Histochem. Cytobiol., 24*(10) :65-70.
13. Tojo M *et al*. (1988), Isolation and characterization of a capsular polysaccharide adhesion from *Staphylococcus epidermidis., J. Infect. Dis., 157*:713-722.
14. Khoury AE and Costerton JW (1991), Bacterial biofilms in nature and disease. *In:* Dialogues in Pediatric Urology, *14*:1-8.
15. Nickel JC *et al.* (1985), Tobramycin Resistance of *Rseudomonas aeruginosa* Cells Growing as a Biofilm on Urinary Catheter Material., *Antimicrobial Agents and Chemotherapy, 27*:619-624.
16. Evans RC and Holmes CJ (1987), Effect of Vancomycin Hydrochloride on *Staphylococcus epidermidis* Biofilm Associated with Silicone Elastomer., *Antimicrobial Agents and Chemotherapy, 31*:889-894.
17. Solomon *et al.* (1987), *J. Controlled Release, 6*:343-352.
18. U.S. Patent No. 4,442,133.
19. U.S. Patent No. 4,678,660.
20. U.S. Patent No. 4,749,585.
21. U.S. Patent No. 4,895,566.
22. U.S. Patent No. 4,917,686.
23. U.S. Patent No. 4,107,121.
24. U.S. Patent No. 5,013,306.
25. U.S. Patent No. 4,952,419.
26. Sherertz *et al*. (1989), *Antimicrob Agents Chemother,* 33:114-118.

## Claims

1. An implantable medical device resistant to microbial growth and coated with a composition comprising EDTA and minocycline.

2. A catheter resistant to glycocalyx formation, said catheter being coated with a composition comprising EDTA and minocycline.

3. The medical device of claim 1 or the catheter of claim 2 obtainable by a process of:
obtaining a medical device or catheter; and
treating the device or catheter surface with a preparation comprising minocycline and EDTA.

4. The medical device or catheter of claim 3 wherein the preparation comprises between 10 to 100 mg/ml minocycline and between 10 to 100 mg/ml EDTA.

5. The medical device or catheter of claim 4 wherein the preparation includes 60 mg/ml minocycline and 60 mg/ml EDTA.

6. The medical device or catheter of claim 3 wherein the device or catheter is coated with a surfactant.

7. The medical device or catheter of claim 6 wherein the surfactant is tridodecylmethyl ammonium chloride.

8. The medical device or catheter of claim 6 wherein the surfactant is benzalkonium chloride.

9. The medical device of claim 1 or 3 or the catheter of claim 2 or 3 further defined as a central venous catheter, a peripheral intravenous catheter, an arterial catheter, a Swan-Ganz catheter, a hemodialysis catheter, an umbilical catheter, a percutaneous nontunneled silicone catheter, a cuffed tunneled central venous catheter or a subcutaneous central venous port.

10. The medical device of claim 1 or 3 or the catheter of claim 2 or 3 further defined as a urinary catheter or a peritoneal catheter.

11. A method for preparing a glycocalyx-resistant medical device or catheter comprising coating the device or catheter with a composition comprising 60 mg/ml EDTA and 60 mg/ml minocycline and allowing the coating to dry on the surface of the device or catheter.

12. The method of claim 11 wherein the device or catheter is an indwelling catheter.

13. The method of claim 11 wherein the device or catheter is a central venous catheter or a triple lumen catheter.

14. A method for preparing a biofilm-resistant medical device using a pharmaceutical preparation of minocycline and EDTA, comprising:
obtaining a medical device;
preparing preparation including at least 10 mg/ml minocycline and at least 10 mg/ml EDTA; and
treating the device surface with the pharmaceutical preparation to allow the formation of a film of the preparation on the device surface.

15. The method of claim 14 wherein the medical device is a catheter.

16. The method of claim 14 wherein the biofilm is a microbial glycocalyx.

17. The method of claim 14 wherein the biofilm is a polysaccharide-rich glycocalyx.

18. The method of claim 14 wherein the biofilm is a staphylococcal glycocalyx.

19. The method of claim 14 wherein the pharmaceutical preparation includes between 10 to 100 mg/ml minocycline and between 10 to 100 mg/ml EDTA.

20. The method of claim 14 wherein the pharmaceutical preparation includes 60 mg/ml minocycline and 60 mg/ml EDTA.

21. A composition comprising minocycline and at least 10 mg/ml EDTA.

22. The composition of claim 21 comprising between 10 mg/ml and 100 mg/ml EDTA.

23. The composition of claim 21 comprising between 0.001 mg/ml and 100 mg/ml minocycline.

24. The composition of claim 23 comprising 30 mg/ml EDTA and 30 mg/ml minocycline.

25. The composition of claim 23 comprising 60 mg/ml EDTA and 60 mg/ml minocycline.

26. The compostition of claim 21 comprising 3 mg/ml minocycline and 30 mg/ml EDTA.

27. The composition of claim 26 in a saline carrier solution.

28. The composition of any of claims 21 to 27 for use as a medicinal product.

29. Use of a composition according to any of claims 21 to 27 for the manufacture of a medicinal product for inhibiting or eliminating microbial glycocalyx formation at a catheter.

30. Use of a minocycline solution comprising between 0.001 to 100 mg/ml minocycline for the manufacture of a medicinal product for preventing catheter infection.

31. The use according to claim 28 wherein the solution comprises 3 mg/ml minocycline.

## Patentansprüche

1. Implantierbare medizinische Vorrichtung, die resistent ist gegen ein mikrobielles Wachstum und die mit einer Zusammensetzung beschichtet ist, die EDTA und Minocyclin umfasst.

2. Katheter, der resistent ist gegen eine Glykokalyx-Bildung und der mit einer Zusammensetzung beschichtet ist, die EDTA und Minocyclin umfasst.

3. Medizinische Vorrichtung nach Anspruch 1 oder Katheter nach Anspruch 2, erhältlich durch ein Verfahren, wobei
- eine medizinische Vorrichtung oder ein Katheter erhalten wird, und
- die Oberfläche der Vorrichtung oder des Katheters mit einer Zubereitung behandelt wird, die Minocyclin und EDTA umfasst.

4. Medizinische Vorrichtung oder Katheter nach Anspruch 3, wobei die Zubereitung zwischen 10 und 100 mg/ml Minocyclin und zwischen 10 und 100 mg/ml EDTA umfasst.

5. Medizinische Vorrichtung oder Katheter nach Anspruch 4, wobei die Zubereitung 60 mg/ml Minocyclin und 60 mg/ml EDTA umfasst.

6. Medizinische Vorrichtung oder Katheter nach Anspruch 3, wobei die Vorrichtung oder der Katheter mit einem oberflächenaktiven Stoff beschichtet ist.

7. Medizinische Vorrichtung oder Katheter nach Anspruch 6, wobei der oberflächenaktive Stoff Tridodecylmethylammoniumchlorid ist.

8. Medizinische Vorrichtung oder Katheter nach Anspruch 6, wobei der oberflächenaktive Stoff Benzalkoniumchlorid ist.

9. Medizinische Vorrichtung nach Anspruch 1 oder 3 oder Katheter nach Anspruch 2 oder 3, ferner definiert als ein zentraler Venenkatheter, ein peripherer intravenöser Katheter, ein arterieller Katheter, ein Einschwemmkatheter, ein Hämodialysekatheter, ein Nabelkatheter, ein perkutaner nicht-durchtunnelter Silikonkatheter, ein durchtunnelter zentraler venöser Manschettenkatheter oder ein subkutaner zentraler Venenport.

10. Medizinische Vorrichtung nach Anspruch 1 oder 3 oder Katheter nach Anspruch 2 oder 3, ferner definiert als ein Urinkatheter oder ein Peritonealkatheter.

11. Verfahren zur Herstellung einer Glykokalyx-resistenten, medizinischen Vorrichtung oder Katheters, welches die Beschichtung der Vorrichtung oder des Katheters mit einer Zusammensetzung, die 60 mg/ml EDTA und 60 mg/ml Minocyclin umfasst, und die Trocknung der Beschichtung auf der Oberfläche der Vorrichtung oder des Katheters umfasst.

12. Verfahren nach Anspruch 11, wobei die Vorrichtung oder der Katheter ein Dauerkatheter ist.

13. Verfahren nach Anspruch 11, wobei die Vorrichtung oder der Katheter ein zentraler Venenkatheter oder ein dreifacher Lumenkatheter ist.

14. Verfahren zur Herstellung einer Biofilm-resistenten, medizinischen Vorrichtung unter Verwendung einer pharmazeutischen Zubereitung aus Minocyclin und EDTA, welches umfasst:
- den Erhalt einer medizinischen Vorrichtung,
- die Herstellung einer Zubereitung, die wenigstens 10 mg/ml Minocyclin und wenigstens 10 mg/ml EDTA umfasst, und
- die Behandlung der Oberfläche der Vorrichtung mit der pharmazeutischen Zubereitung, um die Bildung eines Films der Zubereitung auf der Oberfläche der Vorrichtung zu ermöglichen.

15. Verfahren nach Anspruch 14, wobei die medizinische Vorrichtung ein Katheter ist.

16. Verfahren nach Anspruch 14, wobei der Biofilm eine mikrobielle Glykokalyx ist.

17. Verfahren nach Anspruch 14, wobei der Biofilm eine Polysaccharid-reiche Glykokalyx ist.

18. Verfahren nach Anspruch 14, wobei der Biofilm eine Staphylokokken-Glykokalyx ist.

19. Verfahren nach Anspruch 14, wobei die pharmazeutische Zubereitung zwischen 10 und 100 mg/ml Minocyclin und zwischen 10 und 100 mg/ml EDTA umfasst.

20. Verfahren nach Anspruch 14, wobei die pharmazeutische Zubereitung 60 mg/ml Minocyclin und 60 mg/ml EDTA umfasst.

21. Zusammensetzung, umfassend Minocyclin und wenigstens 10 mg/ml EDTA.

22. Zusammensetzung nach Anspruch 21, die zwischen 10 mg/ml und 100 mg/ml EDTA umfasst.

23. Zusammensetzung nach Anspruch 21, die zwischen 0,001 mg/ml und 100 mg/ml Minocyclin umfasst.

24. Zusammensetzung nach Anspruch 23, die 30 mg/ml EDTA und 30 mg/ml Minocyclin umfasst.

25. Zusammensetzung nach Anspruch 23, die 60 mg/ml EDTA und 60 mg/ml Minocyclin umfasst.

26. Zusammensetzung nach Anspruch 21, die 3 mg/ml Minocyclin und 30 mg/ml EDTA umfasst.

27. Zusammensetzung nach Anspruch 26 in einer salinischen Trägerlösung.

28. Zusammensetzung nach einem der Ansprüche 21 bis 27 zur Verwendung als ein medizinisches Produkt.

29. Verwendung einer Zusammensetzung nach einem der Ansprüche 21 bis 27 für die Herstellung eines medizinischen Produktes zur Hemmung oder Eliminierung einer mikrobiellen Glykokalyx-Bildung an einem Katheter.

30. Verwendung einer Minocyclinlösung, die zwischen 0,001 bis 100 mg/ml Minocyclin umfasst, für die Herstellung eines medizinischen Produktes zur Verhinderung einer Katheterinfektion.

31. Verwendung nach Anspruch 28, wobei die Lösung 3 mg/ml Minocyclin umfasst.

## Revendications

1. Dispositif médical pouvant être implanté résistant à la croissance microbienne et revêtu d'une composition comprenant de l'EDTA et de la minocycline.

2. Cathéter résistant à la formation de glycocalyx, ledit cathéter étant revêtu d'une composition comprenant de l'EDTA et de la minocycline.

3. Dispositif médical selon la revendication 1 ou cathéter selon la revendication 2, pouvant être obtenus par un procédé consistant à :
- obtenir un dispositif médical ou un cathéter ; et
- traiter la surface du dispositif ou du cathéter avec une préparation comprenant la minocycline et de l'EDTA.

4. Dispositif médical ou cathéter selon la revendication 3, dans lequel la préparation comprend entre 10 et 100 mg/ml de minocycline et entre 10 et 100 mg/ml d'EDTA.

5. Dispositif médical ou cathéter selon la revendication 4, dans lequel la préparation comprend 60 mg/ml de minocycline et 60 mg/ml d'EDTA.

6. Dispositif médical ou cathéter selon la revendication 3, dans lequel le dispositif ou cathéter est revêtu d'un tensio-actif.

7. Dispositif médical ou cathéter selon la revendication 6, dans lequel le tensio-actif est le chlorure de tridodécylméthylammonium.

8. Dispositif médical ou cathéter selon la revendication 6, dans lequel le tensio-actif est le chlorure de benzalkonium.

9. Dispositif médical selon la revendication 1 ou 3 ou cathéter selon la revendication 2 ou 3, défini de plus en tant que cathéter veineux central, cathéter intraveineux périphérique, cathéter artériel, cathéter de Swan-Ganz, cathéter d'hémodialyse, cathéter ombilical, cathéter de silicone percutané non tunnellisé, cathéter veineux central avec ballonnet tunnellisé ou accès veineux central sous-cutané.

10. Dispositif médical selon la revendication 1 ou 3 ou cathéter selon la revendication 2 ou 3, défini de plus en tant que cathéter urinaire ou cathéter péritonéal.

11. Procédé pour préparer un dispositif médical résistant au glycocalyx ou cathéter consistant à revêtir le dispositif ou le cathéter avec une composition comprenant 60 mg/ml d'EDTA et 60 mg/ml de minocycline et à laisser sécher le revêtement sur la surface du dispositif ou du cathéter.

12. Procédé selon la revendication 11, dans lequel le dispositif ou cathéter est un cathéter à demeure.

13. Procédé selon la revendication 11, dans lequel le dispositif ou cathéter est un cathéter veineux central ou un cathéter à triple lumière.

14. Procédé pour préparer un dispositif médical résistant au biofilm utilisant une préparation pharmaceutique de minocycline et d'EDTA, consistant à :
- obtenir un dispositif médical ;
- préparer une préparation comprenant au moins 10 mg/ml de minocycline et au moins 10 mg/ml d'EDTA ; et
- traiter la surface du dispositif avec la préparation pharmaceutique pour permettre la formation d'un film de la préparation sur la surface du dispositif.

15. Procédé selon la revendication 14, dans lequel le dispositif médical est un cathéter.

16. Procédé selon la revendication 14, dans lequel le biofilm est un glycocalyx microbien.

17. Procédé selon la revendication 14, dans lequel le biofilm est un glycocalyx riche en polysaccharide.

18. Procédé selon la revendication 14, dans lequel le biofilm est un glycocalyx de staphylocoque.

19. Procédé selon la revendication 14, dans lequel la préparation pharmaceutique comprend de 10 à 100 mg/ml de mincocycline et de 10 à 100 mg/ml d'EDTA.

20. Procédé selon la revendication 14, dans lequel la préparation pharmaceutique comprend 60 mg/ml de minocycline et 60 mg/ml d'EDTA.

21. Composition comprenant de la minocycline et au moins 10 mg/ml d'EDTA.

22. Composition selon la revendication 21, comprenant entre 10 mg/ml et 100 mg/ml d'EDTA.

23. Composition selon la revendication 21, comprenant entre 0,001 mg/ml et 100 mg/ml de minocycline.

24. Composition selon la revendication 23, comprenant 30 mg/ml d'EDTA et 30 mg/ml de minocycline.

25. Composition selon la revendication 23, comprenant 60 mg/ml d'EDTA et 60 mg/ml de minocycline.

26. Composition selon la revendication 21, comprenant 3 mg/ml de minocycline et 30 mg/ml d'EDTA.

27. Composition selon la revendication 26 dans une solution porteuse saline.

28. Composition selon l'une quelconque des revendications 21 à 27, pour une utilisation en tant que produit médical.

29. Utilisation d'une composition selon l'une quelconque des revendications 21 à 27, pour la fabrication d'un produit médical pour inhiber ou éliminer la formation de glycocalyx microbien sur un cathéter.

30. Utilisation d'une solution de minocycline comprenant entre 0,001 et 100 mg/ml de minocycline pour la fabrication d'un produit médical pour empêcher l'infection du cathéter.

31. Utilisation selon la revendication 28, dans laquelle la solution comprend 3 mg/ml de minocycline.
